# EUROPEAN PATENT APPLICATION

(11) **EP 4 706 736 A2**
(43) Date of publication of application: **11.03.2026**
(21) Application number: 26152385.6
(22) Date of filing: 29.03.2023
(51) Int. Cl.: A61M 25/09

(54) **DEVICE FOR MANUALLY LOCKING AN INSERTED LINE DRAW DEVICE TO PREVENT PREMATURE RETRACTION**

(30) Priority: 31.03.2022 US 202263326001 P
(62) Divisional of application: 23781724.2
(71) Applicant: Becton, Dickinson and Company, Franklin Lakes, NJ 07417 (US)
(72) Inventor: HARDING, Weston, F., Lehi, UT 84043 (US); BLANCHARD, Curtis, H., Herriman, UT 84096 (US); SCHERICH, Megan, S., Salt Lake City, UT 84115 (US); LACKEY, John, M., West Valley City, UT 84119 (US); BAILEY, Lisa, Salt Lake City, UT 84106 (US); HOPWOOD, Benjamin, P., West Valley City, UT 84128 (US); BOUD, Adam, J., Bluffdale, UT 84065 (US)
(74) Representative: dompatent

(57) **Abstract**

A fluid transfer device including a housing, a distal introducer portion configured to penetrate a needleless access connector of a vascular access device, and a wheel member rotatable with respect to the housing and operably coupled to a probe to advance and retract the probe through the distal introducer portion. The fluid transfer device also includes a connector member positioned proximate the distal introducer portion, wherein the connector member includes a pair of opposing distal clip portions, a pair of opposing proximal clip portions, and a locking member. The locking member is operably coupled to the wheel member and is configured to prevent actuation of at least a portion of the connector member when the wheel member is rotated to a first position and to allow actuation at least a portion of the connector member when the wheel member is rotated to a second position.

## Description

### CROSS-REFERENCE TO RELATED APPLICATION

The present application aspects priority to United States Provisional Application No. 63/326,001, entitled "Device for Manually Locking an Inserted Line Draw Device to Prevent Premature Retraction", filed March 31, 2022, the entire disclosure of which is hereby incorporated by reference in its' entirety.

### BACKGROUND OF THE INVENTION

### Field of the Invention

The present disclosure generally relates to blood collection devices and related assemblies, systems, and methods for use in blood collection via an existing vascular access device (VAD) such as, e.g., a peripheral intravenous catheter (PIVC). Specifically, the blood collection devices of the present disclosure are configured to prevent premature retraction and/or removal of the device.

### Description of Related Art

A vascular access device (VAD) such as a catheter is commonly used to infuse fluids into vasculature of a patient. For example, the catheter may be used for infusing normal saline solution, various medicaments, or total parenteral nutrition. In some instances, the VAD may be an over-the-needle peripheral intravenous catheter (PIVC).

In addition to infusion, VADs may also be used for withdrawing blood from the patient, although they are not typically designed and optimized for such purposes. In view of various challenges related to blood extraction from VADs, fluid transfer devices have been developed to mitigate the possibility of catheter collapse, reduced blood flow due to debris built up on or within the catheter, etc. Examples of such fluid transfer devices are shown and described in U.S. Patent Application Publication No. 2021/0290905 A1, which is incorporated herein by reference in its entirety.

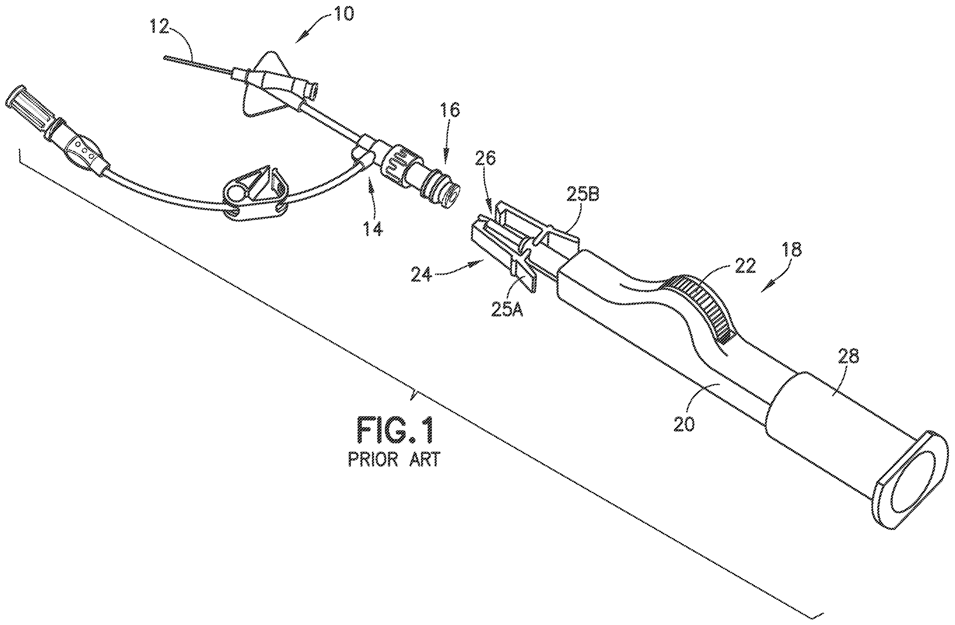

FIG. 1 illustrates a fluid transfer device 18 similar to those shown and described in U.S. Patent Application Publication No. 2021/0290905 A1. Fluid transfer device 18 is configured to be releasably coupled to an adapter 14 having a needleless access connector 16, with the adapter 14 coupled to a catheter adapter 10 having a catheter 12 extending therefrom and configured for vascular access. The fluid transfer device 18 includes a housing 20 having a connector 24 and a distal introducer portion 26 positioned at a distal end portion, as well as vacuum tube receiver 28 positioned at a proximal end portion, with the vacuum tube receiver 28 configured to accommodate a blood collection device such as, e.g., a BD VACUTAINER^{®} blood collection tube in order to collect a blood sample from the patient via the VAD. While not shown, the fluid transfer device 18 includes an elongated probe (e.g., a nickel titanium wire) that can be selectively advanced in a distal direction through the adapter 14, the catheter adapter 10, and beyond the distal tip of the catheter 12, with probe providing a fluid channel to allow for blood draw into a blood collection device within vacuum tube receiver 28. Advancement of the probe of fluid transfer device 18 is controlled by a wheel 22 rotatable within the housing 20, which enables a clinician to use his or her thumb to rotate the wheel 22 in a first direction to advance the probe, and in a second, opposite direction to retract the probe.

Prior to deployment of the probe, the fluid transfer device 18 is coupled to the adapter 14, with the distal introducer portion 26 being inserted through a septum in the needleless access connector 16. Upon sufficient distal insertion of the distal introducer portion 26 within the needleless access connector 16, the connector 24, in the form of lever lock clips, is configured to clip onto the adapter 14 to secure the fluid transfer device 18 in place. Once secured, the probe may be advanced from the fluid transfer device 18 by way of rotation of the wheel 22.

Typically, the probe is retracted into the fluid transfer device 18 after blood collection via rotation of the wheel 22 in the opposite direction, at which point the connector 24 may be decoupled from the adapter 14 by pinching proximal clip portions 25A, 25B to release the "alligator clip" distal connection of connector 24 from the adapter 14. However, in some instances, a clinician or other user may decouple the connector 24 from the adapter 14 and retract the distal introducer portion 26 while the probe is still in its advanced state within the adapter 14, catheter adapter 10, and/or catheter 12. Such premature retraction and/or removal of the fluid transfer device 18 may result in undesirable exposure to blood or other bodily fluids, and/or may result in damage to the probe.

The subject matter aspected herein is not limited to embodiments that solve any disadvantages or that operate only in environments such as those described above. Rather, this background is only provided to illustrate one example technology area where some implementations described herein may be practiced.

### SUMMARY OF THE INVENTION

In accordance with an aspect of the present disclosure a fluid transfer device for delivery of a probe to a patient's vascular system is disclosed. The fluid transfer device includes a housing, a distal introducer portion positioned at a distal end portion of the housing and configured to penetrate a needleless access connector of a vascular access device, and a wheel member rotatable with respect to the housing, wherein the wheel member is operably coupled to the probe to advance and retract the probe through the distal introducer portion based on direction of rotation of the wheel member. The fluid transfer device also includes a connector member positioned proximate the distal introducer portion and configured for releasable connection to at least one surface of the needleless access connector, wherein the connector member includes a pair of opposing distal clip portions and a pair of opposing proximal clip portions. The fluid transfer device further includes a locking member, wherein the locking member is operably coupled to the wheel member and is configured to prevent actuation of at least one of the pair of opposing proximal clip portions when the wheel member is rotated to a first position and to allow actuation of at least one of the pair of opposing proximal clip portions when the wheel member is rotated to a second position.

In some embodiments, the wheel member includes a clocking member, and the locking member is operably coupled to the clocking member.

In some embodiments, the locking member includes an arm coupled to the clocking member.

In some embodiments, one end of the arm includes a post configured to ride within a cam groove formed in the clocking member.

In some embodiments, the locking member includes a ring member longitudinally slidable over at least a portion of the housing.

In some embodiments, the locking member includes a biasing member configured to bias the locking member in one direction.

In some embodiments, the fluid transfer device further includes a rack member and a pinion gear, wherein the pinion gear is operably coupled to the wheel member and the rack member is coupled to the locking member so as to longitudinally displace the locking member based upon rotational direction of the wheel member.

In some embodiments, the locking member includes at least one tab, and the at least one tab is displaceable by a wire operably coupled to the wheel member.

In some embodiments, the locking member includes at least one proximal arm member, and the wheel member includes at least one slot provided on a radial surface of the wheel member, wherein the at least one slot is sized and configured to accommodate at least a portion of the at least one proximal arm member.

In some embodiments, the locking member further includes at least one living hinge coupled to at least one of the pair of opposing proximal clip portions on a first end and to the at least one proximal arm member on a second end.

In some embodiments, the locking member further includes a ramped distal member.

In some embodiments, the locking member is manually displaceable by a user.

In some embodiments, the locking member includes a distal blocking portion and a proximal button portion, wherein the proximal button portion is configured as a user interface.

According to another aspect of the present disclosure, a fluid transfer device for needle-free delivery of a probe to a patient's vascular system is disclosed. The fluid transfer device includes a housing, a distal introducer portion positioned at a distal end portion of the housing and configured to penetrate a needleless access connector of a vascular access device, and a wheel member rotatable with respect to the housing, wherein the wheel member is operably coupled to the probe to advance and retract the probe through the distal introducer portion based on direction of rotation of the wheel member. The fluid transfer device also includes a connector member positioned proximate the distal introducer portion and configured for releasable connection to at least one surface of the needleless access connector and a locking member, wherein the locking member is configured to prevent disconnection of the connector member from the needleless access connector when the wheel member is rotated to a first position and to allow disconnection of the connector member when the wheel member is rotated to a second position.

In some embodiments, the locking member includes a sleeve member.

In some embodiments, the sleeve member is configured to extend over at least a portion of the connector member when the wheel member is rotated to the first position.

In some embodiments, at least a portion of the locking member is configured to extend under at least a portion of the connector member when the wheel member is in the first position.

In some embodiments, the wheel member further includes a pair of opposing slots provided on opposing sides of the wheel member, and the locking member includes a pair of opposing inner projections provided on a proximal portion of the connector member, the pair of opposing inner projections configured to engage the pair of opposing slots when the wheel member is rotated to the second position.

In some embodiments, the device further includes a projecting member extending from at least one side of the probe, wherein the projecting member is configured to enable movement of the locking member based upon a position of the probe within the housing.

In some embodiments, the locking member is manually displaceable by a user, and the locking member includes a distal blocking portion and a proximal button portion, wherein the proximal button portion is configured as a user interface.

It is to be understood that both the foregoing general description and the following detailed description are examples and explanatory and are not restrictive of the invention, as aspected. It should be understood that the various embodiments are not limited to the arrangements and instrumentality shown in the drawings. It should also be understood that the embodiments may be combined, or that other embodiments may be utilized and that structural changes, unless so aspected, may be made without departing from the scope of the various embodiments of the present invention. The following detailed description is, therefore, not to be taken in a limiting sense.

### BRIEF DESCRIPTION OF THE DRAWINGS

Example embodiments will be described and explained with additional specificity and detail through the use of the accompanying drawings in which:
FIG. 1 is a perspective view of a prior art vascular access configuration and fluid transfer device;
FIG. 2 is a side cross-sectional view of a fluid transfer device in accordance with an aspect of the present disclosure;
FIG. 3 is a top cross-sectional view of a fluid transfer device in accordance with another aspect of the present disclosure;
FIG. 4 is a side cross-sectional view the fluid transfer device of FIG. 3;
FIG. 5 is a top cross-sectional view of a fluid transfer device in accordance with another aspect of the present disclosure;
FIG. 6 is a side cross-sectional view the fluid transfer device of FIG. 5;
FIG. 7 is a side view of a clocking member with external cam for use with a wheel member in accordance with an aspect of the present disclosure;
FIG. 8 is an end view of the clocking member with external cam of FIG. 7;
FIG. 9 is a side cross-sectional view of a fluid transfer device in accordance with another aspect of the present disclosure in a first configuration;
FIG. 10 is a side cross-sectional view of the fluid transfer device of FIG. 9 in a second configuration;
FIG. 11 is a top cross-sectional view of a fluid transfer device in accordance with another aspect of the present disclosure;
FIG. 12 is a side view of a wheel member and rack-and-pinion configuration for use with the fluid transfer device of FIG. 11;
FIG. 13 is a top view of a fluid transfer device in accordance with another aspect of the present disclosure;
FIG. 14 is an perspective cross-sectional view of the fluid transfer device of FIG. 13;
FIG. 15 is a side view of a wheel member in accordance with an aspect of the present disclosure;
FIG. 16 is a partial side cross-sectional view of a fluid transfer device in accordance with another aspect of the present disclosure;
FIG. 17 is a perspective view of a wire member and tab configuration of the fluid transfer device of FIG. 16;
FIG. 18 is a side view of a connector member and tab configuration of the fluid transfer device of FIG. 16;
FIG. 19 is a top cross-sectional view of a fluid transfer device in accordance with another aspect of the present disclosure;
FIG. 20 is a side cross-sectional view of a locking member for use with a fluid transfer device in accordance with another aspect of the present disclosure;
FIG. 21 is a side cross-sectional view of another locking member for use with a fluid transfer device in accordance with another aspect of the present disclosure
FIG. 22 is a side cross-sectional view of another locking member for use with a fluid transfer device in accordance with another aspect of the present disclosure;
FIG. 23 is a side cross-sectional view of another locking member for use with a fluid transfer device in accordance with another aspect of the present disclosure;
FIG. 24 is a side view of another locking member for use with a fluid transfer device in accordance with another aspect of the present disclosure;
FIG. 25 is an side view of a ring-shaped locking member for use with a fluid transfer device in accordance with another aspect of the present disclosure;
FIG. 26 is an end view of the ring-shaped locking member of FIG. 25;
FIG. 27 is a partial side cross-sectional view of a fluid transfer device in accordance with another aspect of the present disclosure in a first configuration;
FIG. 28 is a partial side cross-sectional view of the fluid transfer device of FIG. 27 in a second configuration;
FIG. 29 is a top view of a fluid transfer device in accordance with another aspect of the present disclosure in a first configuration;
FIG. 30 is a top view of the fluid transfer device of FIG. 29 in a second configuration;
FIG. 31 is a partial cross-sectional view of a fluid transfer device in accordance with another aspect of the present disclosure in a first configuration;
FIG. 32 is a partial cross-sectional view of a fluid transfer device in accordance with another aspect of the present disclosure in a first configuration;
FIG. 33 is a partial cross-sectional view of the fluid transfer device of FIG. 32 in a second configuration;
FIG. 34 is a partial cross-sectional view of a fluid transfer device in accordance with another aspect of the present disclosure in a first configuration;
FIG. 35 is a partial cross-sectional view of the fluid transfer device of FIG. 34 in a second configuration;
FIG. 36 is a partial cross-sectional view of a fluid transfer device in accordance with another aspect of the present disclosure in a first configuration;
FIG. 37 is a partial cross-sectional view of the fluid transfer device of FIG. 36 in a second configuration;
FIG. 38 is a partial top view of a fluid transfer device in accordance with another aspect of the present disclosure;
FIG. 39 is a top view of a proximal button portion of the fluid transfer device of FIG. 38;
FIG. 40 is a partial cross-sectional view of a fluid transfer device in accordance with another aspect of the present disclosure in a first configuration; and
FIG. 41 is a top view of a fluid transfer device in accordance with another aspect of the present disclosure in a first and second configuration.

### DESCRIPTION OF EMBODIMENTS

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present disclosure.

For the purposes of the description hereinafter, the terms "upper", "lower", "right", "left", "vertical", "horizontal", "top", "bottom", "lateral", "longitudinal", and derivatives thereof shall relate to the invention as it is oriented in the drawings. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a fluid transfer device in preparation for or during use. Similarly, in this application, the terms "in the distal direction" and "distally" mean in the direction toward the connector portion of the fluid transfer device, and the terms "in the proximal direction" and "proximally" mean in the direction opposite the direction of the connector.

While not shown or described herein, it is to be understood that the fluid transfer devices described below may be utilized for blood draw from any suitable vascular access device such as, e.g., the BD NEXIVA^{™} Closed IV Catheter system, the BD CATHENA^{™} Catheter system, the BD VENFLON^{™} Pro Safely Shielded IV Catheter system, the BD NEOFLON^{™} IV Cannula system, the BD INSYTE^{™} AUTOGUARD^{™} BC Shielded IV Catheter system, or another suitable vascular access device.

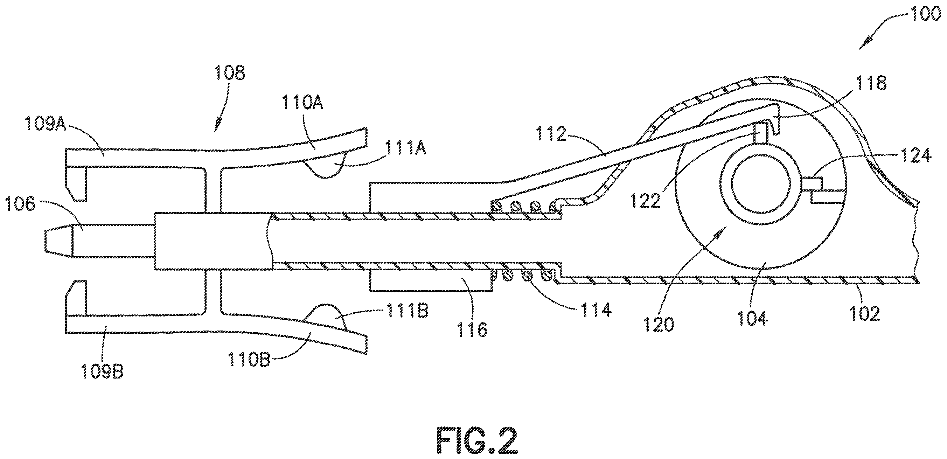

Referring to FIG. 2, a fluid transfer device 100 in accordance with an aspect of the present disclosure is illustrated. The fluid transfer device 100 includes a housing 102 and a wheel member 104. While not shown in FIG. 2, wheel member 104 interacts with an elongated, flexible probe to advance and retract the probe through a distal introducer portion 106 located on a distal end portion of the housing 102. That is, rotation of the wheel member 104 in first direction advances the probe through the distal introducer portion 106 such that the probe can enter the patient's vasculature when fluid transfer device 100 is coupled to a vascular access device. Conversely, rotation of the wheel member 104 in a second, opposite direction retracts the probe through the distal introducer portion 106, ultimately removing the probe from the patient's vasculature.

In some embodiments, the probe is configured as a guidewire (e.g., a nickel titanium wire). However, in other embodiments, probe may be configured as any flexible member or instrument capable of being advanced through a catheter such as, e.g., tubing, a secondary catheter, one or more sensors, obturators, or any combination thereof. Furthermore, in some embodiments, the distal introducer portion 106 is configured as a blunt plastic cannula usable with a needle-free connector of a vascular access device. However, in other embodiments, distal introducer portion 106 may be configured as a male luer connector and/or a needle or cannula capable of accessing a needle-free connector (NFC), a needle access connector (PRN), a luer access port (or female luer), or a septum such as, e.g., a BD NEXIVA^{™} needle septum.

The fluid transfer device 100 also includes a connector member 108 configured for selectively coupling the fluid transfer device 100 to, e.g., a needleless access connector of an adaptor coupled to the vascular access device. Connector member 108 is configured as an alligator clip-type connector, with opposing distal clip portions 109A, 109B allowing for securement of the fluid device 100 to a surface of the needleless access connector. A pair of proximal clip portions 110A, 110B are sized and configured to be pinched or otherwise manipulated by a user. Manipulation of the proximal clip portions 110A, 110B results pivots the distal clip portions 109A, 109B away from one another, thereby resulting in release of the distal clip portions 109A, 109B from engagement with the needleless access connector. In some embodiments, the proximal clip portions 110A, 110B may have respective inner projections 111A, 111B, which may provide a stop or limit to the inward travel of each of the proximal clip portions 110A, 110B with respect to the housing 102. Furthermore, while not shown, in some embodiments, the proximal clip portions 110A, 110B may include a textured surface and/or indicia so as to provide an indication to the user as to the functionality of the connector member 108.

Referring still to FIG. 2, the fluid transfer device 100 further includes an arm member 112, wherein a distal end portion of the arm member 112 is coupled to (or otherwise associated with) a sleeve member 116, and a proximal end portion of the arm member 112 extends to a clocking member 120 rotatable with the wheel member 104. The clocking member 120 includes clocking projections 122, 124, which may interact with a holding portion 118 located at or near a proximal end portion of the arm member 112.

When the wheel member 104 is in a first position such that the probe is retracted within the housing 102, the arm member 112 is held by the clocking projection 122 of clocking member 120 such that the sleeve member 116 is retracted proximally away from the connector member 108. In this position (as shown in FIG. 2), the proximal clip portions 110A, 110B of the connector member 108 are capable of being pinched so as to allow for release the distal clip portions 109A, 109B from engagement with the needleless access connector and, thus, enable removal of the fluid transfer device 100 from engagement with the needleless access connector.

Conversely, if and when the wheel member 104 is rotated toward a second position such that the flexible probe is advanced through the distal introducer portion 106, the clocking projection 122 of clocking member 120 releases the arm member 112, allowing the sleeve member 116 to move distally along the housing 102 in the direction of the connector member 108. In some embodiments, a biasing member 114 (e.g., a spring) may be provided to bias the sleeve member 116 in the distal direction. In this manner, the sleeve member 116 is configured to be positioned substantially below at least the inner projections 111A, 111B of the proximal clip portions 110A, 110B when the wheel member 104 is rotated toward a second position, with the sleeve member 116 being sized and configured to substantially restrict inward motion of the proximal clip portions 110A, 110B when pinched.

By restricting/blocking the inward motion of the proximal clip portions 110A, 110B, the user is unable to release the distal clip portions 109A, 109B from engagement with the needleless access connector and, thus, is unable to remove the fluid transfer device 100 from the needleless access connector when the flexible probe is in an advanced position. However, when the wheel member 104 is rotated back toward the first position to retract the flexible probe, the arm member 112 may again interact with the clocking projection 122 of clocking member 120 in order to overcome the biasing force of the biasing member 114 and pull the sleeve member 116 proximally and away from the connector member 108. When the wheel member 104 is rotated such that the flexible probe is at or near a retracted position within the distal introducer portion 106 and/or the housing 102, the sleeve member 116 is correspondingly positioned away from the connector member 108 such that inward movement of the proximal clip portions 110A, 110B is again possible, thereby allowing for the release of the distal clip portions 109A, 109B from engagement with the needleless access connector and, thus, enabling removal of the fluid transfer device 100 from the needleless access connector when the flexible probe is at or near a retracted position. In this way, premature removal of the fluid transfer device 100 from the needleless access connector can be substantially avoided.

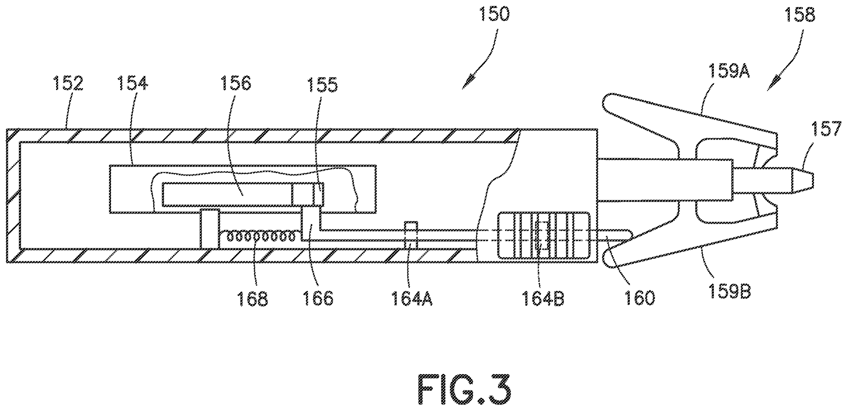

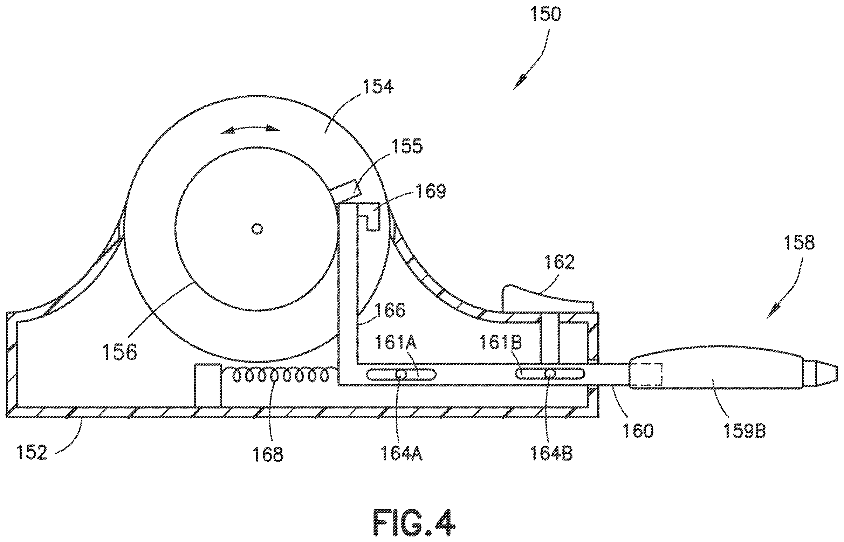

Next, referring to FIGS. 3 and 4, a fluid transfer device 150 in accordance with another aspect of the present disclosure is illustrated. Similar to fluid transfer device 100 described above with respect to FIG. 2, fluid transfer device 150 includes a housing 152 and a wheel member154. Wheel member 154 is configured to interact with an elongated, flexible probe (not shown) to advance and retract the probe through a distal introducer portion 157 located on a distal end portion of the housing 152.

The fluid transfer device 150 also includes a connector member 158 configured for selectively coupling the fluid transfer device 150 to, e.g., a needleless access connector of an adaptor coupled to the vascular access device (not shown). Connector member 158 is configured as an alligator clip-type connector, with opposing distal clip portions allowing for securement of the fluid device 150 to a surface of the needleless access connector, and a pair of proximal clip portions 159A, 159B being sized and configured to be pinched or otherwise manipulated by a user to pivot the distal clip portions away from one another, thereby resulting in release of the connector member 158 from engagement with the needleless access connector. In some embodiments, the proximal clip portions 159A, 159B may have respective inner projections, which may provide a stop or limit to the inward travel of each of the proximal clip portions 159A, 159B with respect to the housing 152. Furthermore, while not shown, in some embodiments, the proximal clip portions 159A, 159B may include a textured surface and/or indicia so as to provide an indication to the user as to the functionality of the connector member 158.

The fluid transfer device 150 further includes an L-shaped arm member movably coupled to the housing 152, the arm member comprising an elongated first portion 160 and a second portion 166 positioned at a proximal end of the first portion 160. A distal end portion of the first portion 160 extends toward the connector member 158, while a proximal end portion of the second portion 166 extends to a clocking member 156 rotatable with the wheel member 154. The clocking member 156 includes a clocking projection 155, which may interact with the proximal end portion of the second portion 166. The first portion 160 further includes slots 161A, 161B formed therethrough, with the slots 161A, 161B sized and configured to accommodate stationary pins 164A, 164B extending from the housing 152. The slots 161A, 161B allow for longitudinal movement of the L-shaped arm member relative to the housing 152, with the pins 164A, 164B providing limits to the longitudinal travel of the L-shaped arm member.

When the wheel member 154 is in a first position such that the probe is retracted within the housing 152, the second portion 166 of the L-shaped arm member may be held by a stop 169 in a retracted position such that the distal end portion of the first portion 160 is retracted proximally away from the connector member 158. In this position, the proximal clip portions 159A, 159B of the connector member 158 are capable of being pinched so as to allow for release the distal clip portions from engagement with the needleless access connector and, thus, enable removal of the fluid transfer device 150 from engagement with the needleless access connector.

On the other hand, if and when the wheel member 154 is rotated toward a second position such that the flexible probe is advanced through the distal introducer portion 157, the clocking projection 155 of clocking member 156 releases the second portion 166 from engagement with the stop 169, allowing the L-shaped arm member, including first portion 160, to move distally along the housing 152 relative to the pins 164A, 164B and in the direction of the connector member 158. In some embodiments, a biasing member 168 (e.g., a spring) may be provided to bias the L-shaped arm member in the distal direction. In this manner, a distal end portion of the first portion 160 of the L-shaped arm member is configured to be positioned substantially below the proximal clip portions 159A, 159B when the wheel member 154 is rotated toward a second position, with the distal end portion of first portion 160 being sized and configured to substantially restrict inward motion of the proximal clip portions 159A, 159B when pinched. While only a single L-shaped arm member is shown in FIGS. 3 and 4, it is to be understood that, in some embodiments, a second arm member may be provided on an opposite side of the housing 152.

In order to pinch the proximal clip portions 159A, 159B to remove the fluid transfer device 150 from engagement with the needleless access connector, the user must first return the probe to a fully or substantially retracted position within the housing 152 by rotating the wheel member 154 back toward the first position. Once the probe is retracted, the user can then manually return the L-shaped arm member to engagement with the stop 169 by sliding a thumb grip 162 provided external to the housing 152 in a proximal direction, which moves the distal end portion of the first portion 160 of the L-shaped arm member proximally away from the connector member 158. This position of the L-shaped arm member is only possible when the probe is substantially or fully retracted and enables the user to pinch the proximal clip portions 159A, 159B to remove the fluid transfer device 150 from engagement with the needleless access connector.

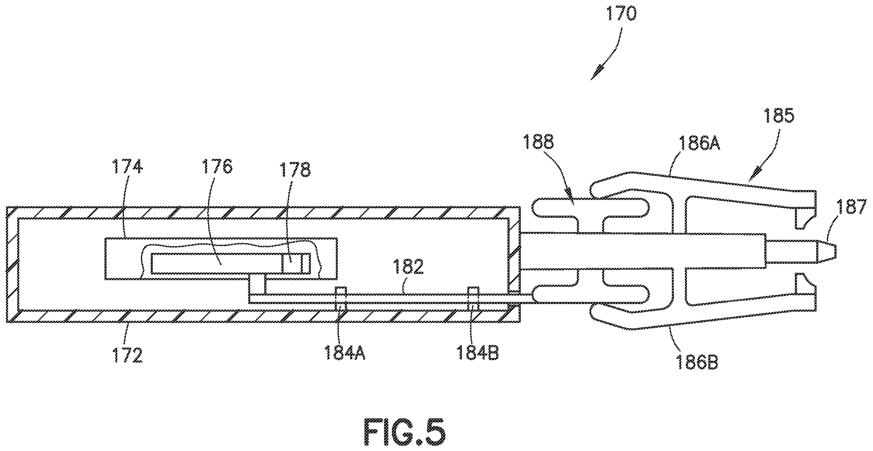

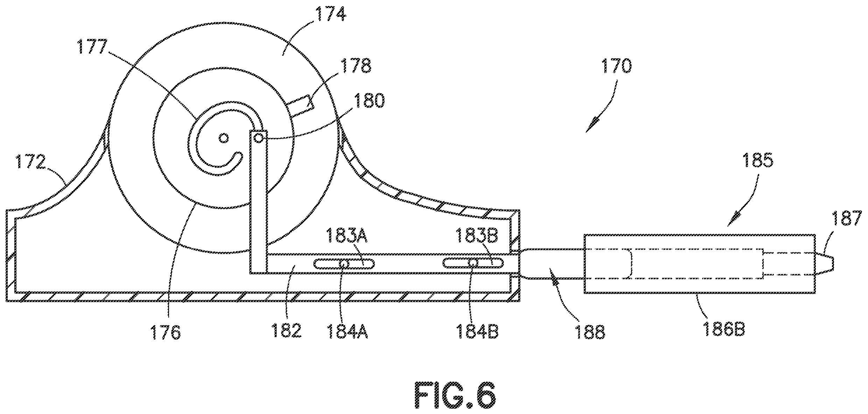

Referring now to FIGS. 5 and 6, a fluid transfer device 170 in accordance with another aspect of the present disclosure is illustrated. The fluid transfer device 170 includes a housing 172 and a wheel member 174. Wheel member 174 is configured to interact with an elongated, flexible probe (not shown) to advance and retract the probe through a distal introducer portion 187 located on a distal end portion of the housing 172. The fluid transfer device 170 further includes a connector member 185 configured for selectively coupling the fluid transfer device 170 to, e.g., a needleless access connector of an adaptor coupled to the vascular access device (not shown). Connector member 185 is configured as an alligator clip-type connector, with opposing distal clip portions allowing for securement of the fluid device 170 to a surface of the needleless access connector, and a pair of proximal clip portions 186A, 186B being sized and configured to be pinched or otherwise manipulated by a user release of the connector member 185 from engagement with the needleless access connector.

The fluid transfer device 170 further includes an L-shaped arm member movable relative to the housing 172, the arm member having an elongated first portion 182 and a second portion 180 positioned at a proximal end of the first portion 182. A distal end portion of the first portion 182 extends toward the connector member 185 and couples to a locking member 188 a distal end thereof. The locking member 188 may be any appropriate shape such as, e.g., ring-shaped, H-shaped, etc.

A proximal end portion of the second portion 180 extends to a clocking member 176 rotatable with the wheel member 174, with the clocking member 176 including a clocking projection 178. Additionally, the clocking member 176 includes a cam groove 177 formed therein, with a pin or other member on the proximal end portion of the second portion 180 configured to interact with the cam groove 177.

The first portion 182 further includes slots 183A, 183B formed therethrough, with the slots 183A, 183B sized and configured to accommodate stationary pins 184A, 184B extending from the housing 172. The slots 183A, 183B allow for longitudinal movement of the L-shaped arm member relative to the housing 172, with the pins 184A, 184B providing limits to the longitudinal travel of the L-shaped arm member.

When the wheel member 174 is in a first position such that the probe is retracted within the distal introducer portion 187 and housing 172, the clocking member 176 is rotated such that second portion 180 of the L-shaped arm member rides along the cam groove 177 to hold the L-shaped arm member in a retracted position. In this retracted position, the locking member 188 is retracted proximally away from the connector member 185, thereby enabling the proximal clip portions 186A, 186B of the connector member 185 to be pinched so as to allow for release the distal clip portions from engagement with the needleless access connector and, thus, enable removal of the fluid transfer device 170 from engagement with the needleless access connector.

Conversely, when the wheel member 174 is rotated toward a second position such that the flexible probe is advanced through the distal introducer portion 187, the second portion 180 of the L-shaped arm member rides along the cam groove 177 of the correspondingly rotating clocking member 176 such that the first portion 182 and locking member 188 move distally along the housing 172 relative to the pins 184A, 168B and in the direction of the connector member 185.

Accordingly, in this "advanced" position of the wheel member 174, the locking member 188 is configured to be positioned substantially below the proximal clip portions 186A, 186B to substantially restrict inward motion of the proximal clip portions 186A, 186B when pinched. In order to pinch the proximal clip portions 186A, 186B to remove the fluid transfer device 170 from engagement with the needleless access connector, the user must first return the probe to a fully or substantially retracted position within the housing 172 by rotating the wheel member 174 back toward the first position. This rotation of the wheel member 174 results in corresponding rotation of the clocking member 176 and, thus, proximal movement of the L-shaped arm member due to the interaction between the second portion 180 and the cam groove 177 of the clocking member 176. Thus, retraction of the probe via wheel member 174 also results in proximal displacement of the locking member 188 away from the connector member 185, which enables the user to pinch the proximal clip portions 186A, 186B to remove the fluid transfer device 170 from engagement with the needleless access connector.

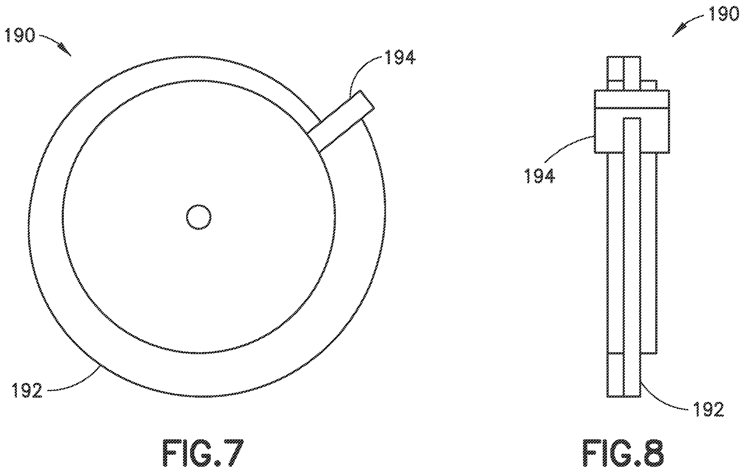

As an alternative to clocking member incorporating a cam groove, as shown and described above with respect to FIGS. 5 and 6, it is to be understood that the clocking member associated with a wheel member may instead utilize an external cam. For example, referring to FIGS. 7 and 8, a clocking member 190 for use with a wheel member (not shown) of a fluid transfer device may incorporate an external cam surface 192 and a clocking projection 194, with the external cam surface 192 capable of interacting with, e.g., an L-shaped arm member to move a connector locking member longitudinally relative to an alligator-clip style connector.

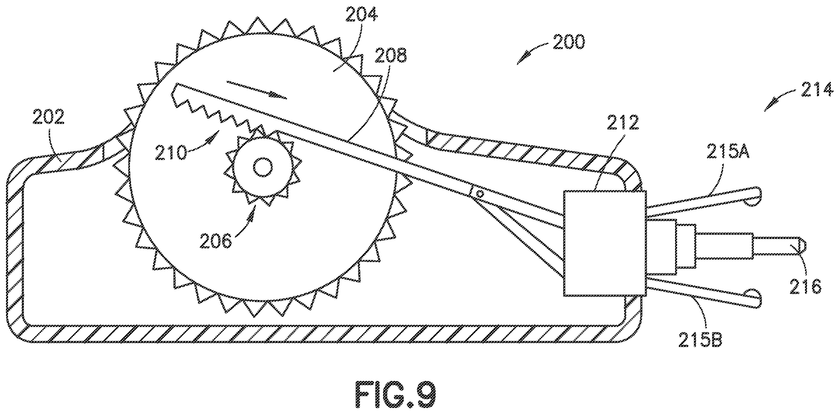

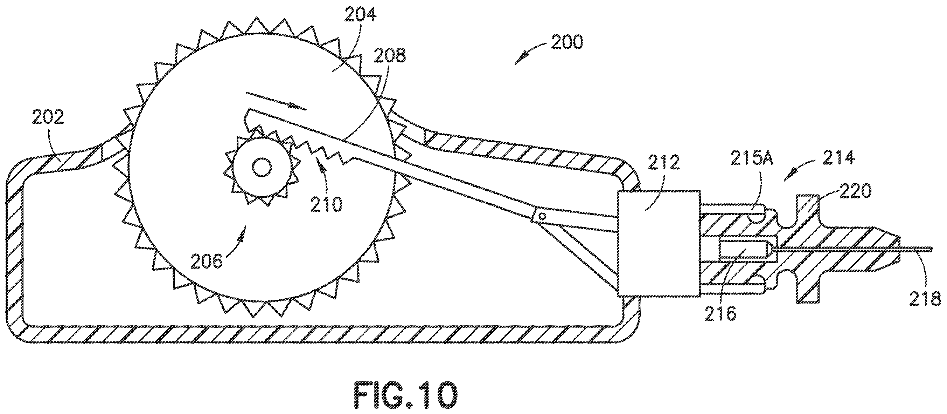

Next, referring to FIGS. 9 and 10, a fluid transfer device 200 in accordance with another aspect of the present disclosure is illustrated. The fluid transfer device 200 includes a housing 202 and a wheel member 204. Wheel member 204 is configured to interact with an elongated, flexible probe 218 (shown in FIG. 10) to advance and retract the probe 218 through a distal introducer portion 216 located on a distal end portion of the housing 202. The fluid transfer device 200 further includes a connector member 214 configured for selectively coupling the fluid transfer device 200 to, e.g., a needleless access connector 220 of an adaptor coupled to the vascular access device (not shown). Connector member 214 may be configured as a collet-type connector, with opposing distal clip portions 215A, 215B allowing for securement of the fluid device 200 to a surface of the needleless access connector 220.

The fluid transfer device 200 further includes an arm member 208 movable relative to the housing 202. The arm member 208 includes a rack portion 210 provided on a proximal surface portion, while a distal portion of the arm member 208 is coupled to a sleeve member 212. The rack portion 210 is configured to engage with a pinion gear member 206 rotatable with the wheel member 204. When the wheel member 204 is in a first position such that the probe is retracted within the distal introducer portion 216 and housing 202 (as shown in FIG. 9), the rack portion 210 of arm member 208 interacts with the pinion gear member 206 such that the arm member 208 and the sleeve member 212 are pulled proximally away from the connector member 214. In this retracted position, the distal clip portions 215A, 215B are able to flex outwardly so as to allow for release the distal clip portions 215A, 215B from engagement with the needleless access connector and, thus, enable removal of the fluid transfer device 200 from engagement with the needleless access connector 220.

However, when the wheel member 204 is rotated toward a second position such that the flexible probe 218 is advanced through the distal introducer portion 216, the rack member 210 rides along the pinion gear member 206 so as to move the arm member 208 and sleeve member 212 distally, with the sleeve member 212 at least partially surrounding the distal clip portions 215A, 215B. Accordingly, in this "advanced" position of the wheel member 204, the sleeve member 212 is configured to substantially prevent outward motion of the distal clip portions 215A, 215B, thereby preventing removal of the fluid transfer device 200 from the needleless access connector 220 when the probe 218 is in an "advanced" position.

To remove the fluid transfer device 200 from engagement with the needleless access connector 220, the user must rotate the wheel member 204 toward a first position to return the probe to a fully or substantially retracted position within the housing 202. This rotation of the wheel member 204 results in corresponding rotation of the pinion gear member 206 and, thus, proximal movement of the arm member 208 and sleeve member 212 due to the interaction between the pinion gear member 206 and the rack portion 210. Thus, retraction of the probe 218 via wheel member 204 also results in proximal displacement of the sleeve member 212 away from the connector member 214, which allows for disengagement and removal of the fluid transfer device 200 from the needleless access connector 220.

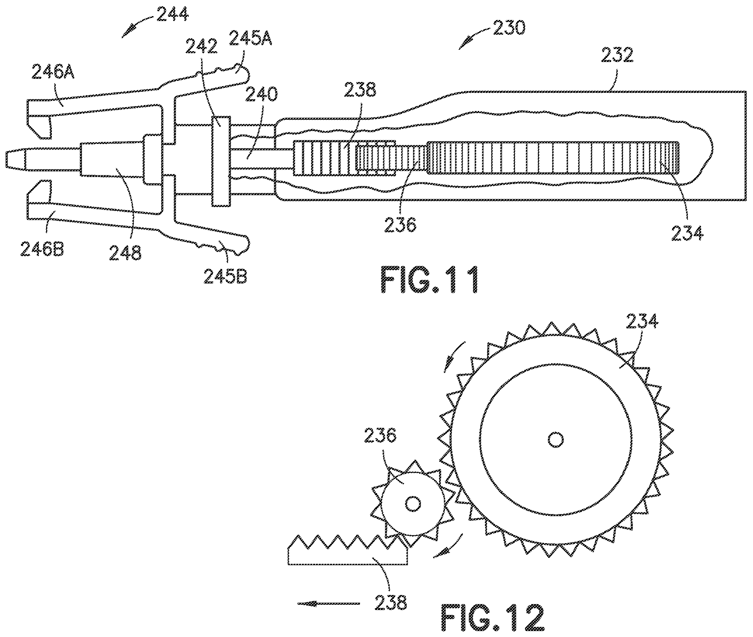

Referring now to FIGS. 11 and 12, a fluid transfer device 230 in accordance with another aspect of the present disclosure is shown. The fluid transfer device 230 includes a housing 232 and a wheel member 234. Wheel member 234 is configured to interact with an elongated, flexible probe (not shown) to advance and retract the probe through a distal introducer portion 248 located on a distal end portion of the housing 232.

The fluid transfer device 230 further includes a connector member 244 configured for selectively coupling the fluid transfer device 230 to, e.g., a needleless access connector of an adaptor coupled to the vascular access device (not shown). Connector member 244 is configured as an alligator clip-type connector, with opposing distal clip portions 246A, 246B allowing for securement of the fluid transfer device 230 to a surface of the needleless access connector, and a pair of proximal clip portions 245A, 245B being sized and configured to be pinched or otherwise manipulated by a user to release the connector member 244 from engagement with the needleless access connector.

The fluid transfer device 230 further includes a locking mechanism comprising a pinion gear member 236, a rack member 238, an arm member 240 coupled to a distal end of the rack member 238, and a lock ring 242. The pinion gear member 236 includes a plurality of teeth configured to engage with corresponding teeth of the wheel member 234, as well as a plurality of teeth on the rack member 238. Accordingly, rotation of the wheel member 234 in a first direction results in rotation of the pinion gear member 236 in a second direction opposite that of the first direction which, in turn, results in longitudinal movement of the rack member 238.

When the wheel member 234 is rotated to a first position such that the probe is retracted within the distal introducer portion 248 and housing 232, the rack portion 238 interacts with the pinion gear member 236 such that the arm member 240 and the lock ring 242 are pulled proximally away from the connector member 244. In this retracted position, the lock ring 242 is retracted proximally away from the connector member 244, thereby enabling the proximal clip portions 245A, 245B of the connector member 244 to be pinched so as to allow for release the distal clip portions 246A, 246B from engagement with the needleless access connector and, thus, enable removal of the fluid transfer device 230 from engagement with the needleless access connector.

Conversely, when the wheel member 234 is rotated toward a second position such that the flexible probe is advanced through the distal introducer portion 248, the rack portion 238 interacts with the pinion gear member 236 such that the arm member 240 and the lock ring 242 move distally toward the connector member 244, with the lock ring 242 ultimately being positioned below the proximal clip portions 245A, 245B. Accordingly, in this "advanced" position of the wheel member 234, the lock ring 242 is configured to substantially prevent inward motion (or pinching) of the proximal clip portions 245A, 245B, thus preventing removal of the fluid transfer device 230 from the needleless access connector when the probe is in an "advanced" position.

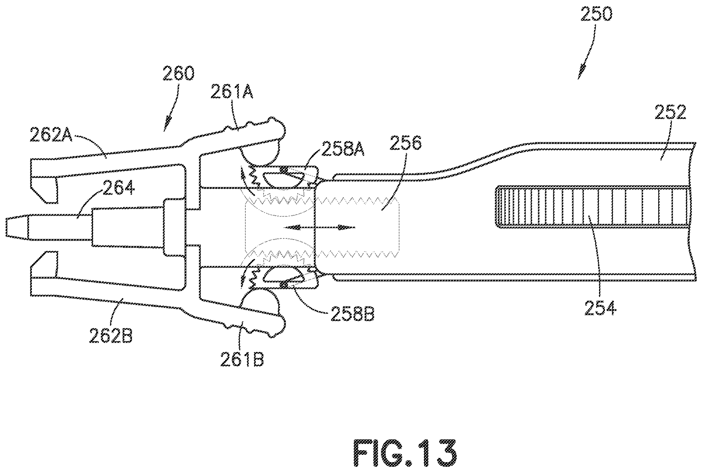

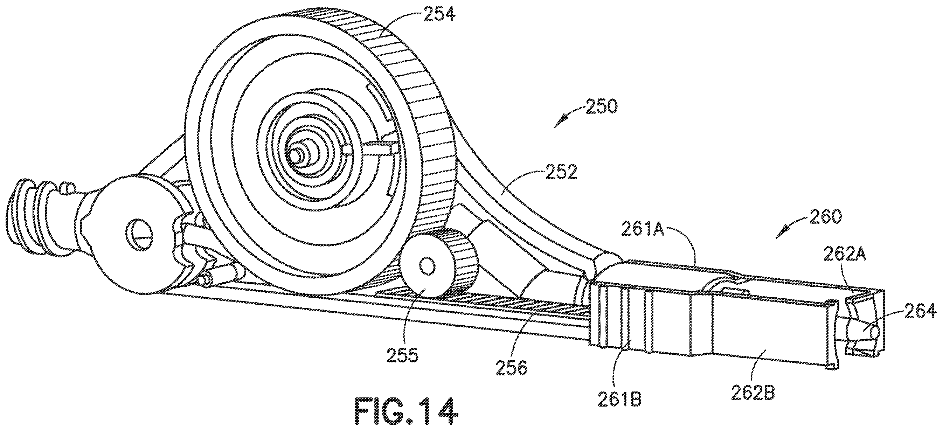

Next, referring to FIGS. 13 and 14, a fluid transfer device 250 in accordance with another aspect of the present disclosure is shown. The fluid transfer device 250 includes a housing 252 and a wheel member 254. Wheel member 254 is configured to interact with an elongated, flexible probe (not shown) to advance and retract the probe through a distal introducer portion 264 located on a distal end portion of the housing 252. A connector member 260 is provided, with the connector member 260 being configured for selectively coupling the fluid transfer device 250 to, e.g., a needleless access connector of an adaptor coupled to the vascular access device (not shown). Connector member 260 is configured as an alligator clip-type connector, with opposing distal clip portions 262A, 262B allowing for securement of the fluid transfer device 250 to a surface of the needleless access connector, and a pair of proximal clip portions 261A, 261B being sized and configured to be pinched or otherwise manipulated by a user to release the connector member 260 from engagement with the needleless access connector.

The fluid transfer device 250 further includes a locking mechanism comprising a pinion gear member 255, a rack member 256, and a pair of segment gear members 258A, 258B positioned laterally and on opposing sides of the rack member 256. The pinion gear member 255 includes a plurality of teeth configured to engage with corresponding teeth of the wheel member 254, as well as a plurality of teeth on the rack member 256. Accordingly, rotation of the wheel member 254 in a first direction results in rotation of the pinion gear member 255 in a second direction opposite that of the first direction which, in turn, results in longitudinal movement of the rack member 256. Furthermore, longitudinal movement of the rack member 256 also results in pivoting-type movement of each of the segment gear members 258A, 258B.

When the wheel member 254 is rotated to a first position such that the probe is retracted within the distal introducer portion 264 and housing 252, the rack portion 256 interacts with the pinion gear member 255 such that the rack portion 256 moves proximally away from the connector member 260, which also causes the segment gear members 258A, 258B to pivot inward and away from the connector member 260. With the segment gear members 258A, 258B pivoted away from the connector member 260, the proximal clip portions 261A, 261B of the connector member 260 are able to be pinched so as to allow for release the distal clip portions 262A, 262B from engagement with the needleless access connector and, thus, enable removal of the fluid transfer device 250 from engagement with the needleless access connector.

On the other hand, when the wheel member 254 is rotated toward a second position such that the flexible probe is advanced through the distal introducer portion 264, the rack portion 256 interacts with the pinion gear member 255 such that the segment gear members 258A, 258B pivot outward and toward the connector member 260, with portions of each of the segment gear members 258A, 258B being positioned below the proximal clip portions 261A, 261B. Accordingly, in this "advanced" position of the wheel member 254, the segment gear members 258A, 258B are configured to substantially prevent inward motion (or pinching) of the proximal clip portions 261A, 261B, thus preventing removal of the fluid transfer device 250 from the needleless access connector when the probe is in an "advanced" position.

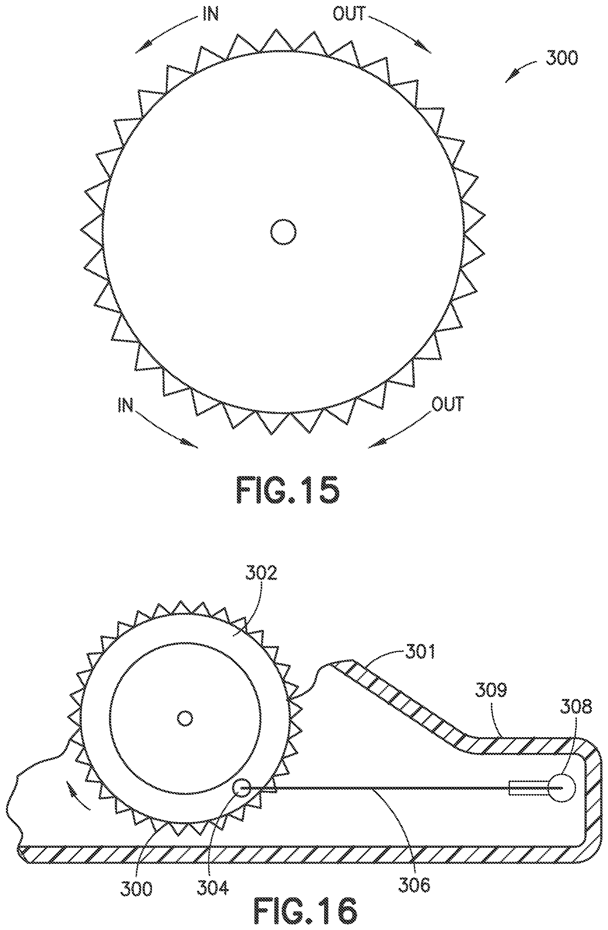

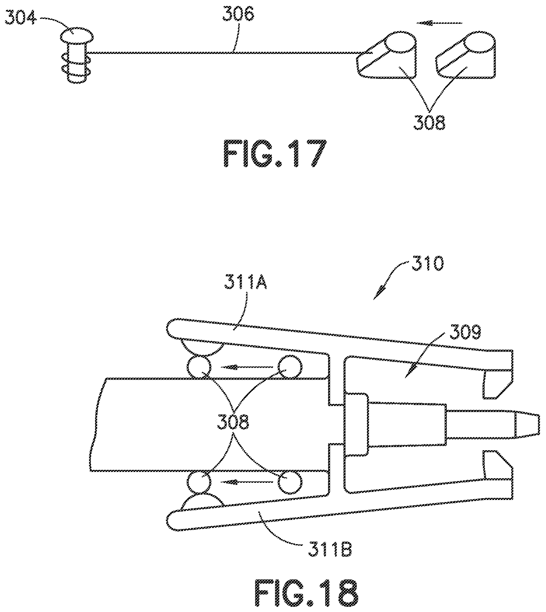

Referring now to FIGS. 15-18, components of a fluid transfer device in accordance with another aspect of the present disclosure are illustrated. The fluid transfer device includes a wheel member 300 disposed within a housing 301 having a distal end portion (or nozzle) 309. As shown in FIG. 18, a connector member 310 may be provided at or near the distal end portion 309 for enabling engagement of the fluid transfer device with a needleless access connector (not shown).

Additionally, wheel member 300 may include a separate rotational member 302 coupled thereto and configured to rotate with the wheel member 300, with rotational member 302 having a post member 304 extending laterally therefrom. A wire 306 is also provided, with a first end of the wire 306 being coupled to the post member 304 and a second end of the wire 306 being coupled to one or more longitudinally slidable tabs 308 positioned at the distal end portion 309 of the housing 301.

When the wheel member 300 is rotated to a first position such that a flexible probe is retracted within the distal end portion 309 and housing 301, the wire 306 is at its fullest extension point, allowing the tab(s) 308 to remain positioned in a distal-most position relative to the distal end portion 309. With the tab(s) 308 in this distal-most position, proximal clip portions 311A, 311B of the connector member 310 are able to be pinched so as to allow for release the distal clip portions 311A, 311B from engagement with the needleless access connector and, thus, enable removal of the fluid transfer device from engagement with the needleless access connector.

However, when the wheel member 300 is rotated toward a second position such that the flexible probe is advanced through distal end portion 309 of the housing 301, the rotational member 302 correspondingly rotates such that the wire 306 is pulled by the post member 304 to displace the tab(s) 308 in a proximal direction, as shown in FIGS. 17 and 18. The tab(s) 308 are ultimately positioned below the proximal clip portions 311A, 311B as the wheel member 300 advances to the second position. Accordingly, in this "advanced" position of the wheel member 300, the tab(s) 308 are configured to substantially prevent inward motion (or pinching) of the proximal clip portions 311A, 311B, thus preventing removal of the fluid transfer device from the needleless access connector when the probe is in an "advanced" position.

While not shown in FIGS. 15-18, it is to be understood that wire 306 may be configured to have sufficient column strength and/or support structure(s) to enable the wire 306 to push the tab(s) 308 back to the distal-most position relative to the distal end portion 309 when the wheel member 300 is rotated back toward the first position to retract the flexible probe.

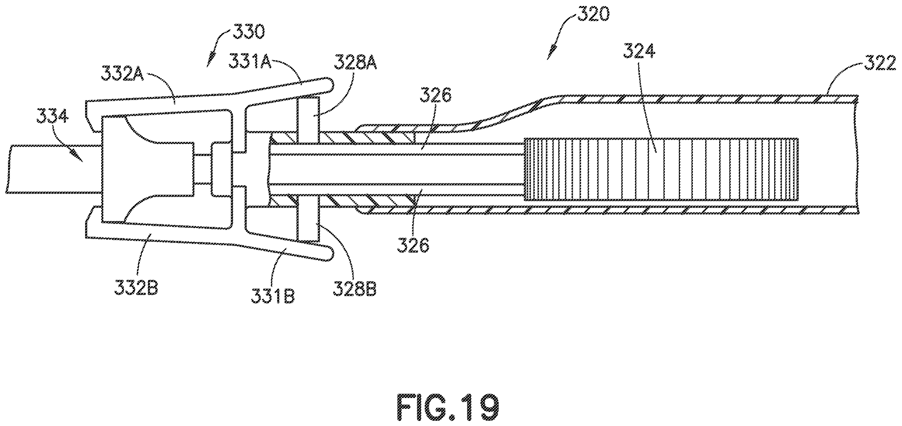

Next, referring to FIG. 19, a fluid transfer device 320 in accordance with another aspect of the present disclosure is shown. The fluid transfer device 320 includes a housing 322 and a rotatable wheel member 324. Wheel member 324 is configured to interact with an elongated, flexible probe (not shown) to advance and retract the probe through a distal introducer portion (not shown) located on a distal end portion of the housing 322. The fluid transfer device 320 further includes a connector member 330 configured for selectively coupling the fluid transfer device 320 to, e.g., a needleless access connector of an adaptor coupled to the vascular access device (not shown). Connector member 330 is configured as an alligator clip-type connector, with opposing distal clip portions 332A, 332B allowing for securement of the fluid transfer device 320 to a surface of the needleless access connector 334, and a pair of proximal clip portions 331A, 331B being sized and configured to be pinched or otherwise manipulated by a user to release the connector member 330 from engagement with the needleless access connector 334.

The fluid transfer device 320 also includes a locking mechanism comprising a linear actuator member 326 and a pair of post members 328A, 328B positioned at or near a distal end portion of the linear actuator member 326. The linear actuator member 326 is configured to be longitudinally displaced along the housing 322 by any appropriate means in concert with rotation of the wheel member 324 such as, e.g., a rack-and-pinion mechanism, a worm gear mechanism, or any other appropriate mechanism.

When the wheel member 320 is rotated to a first position such that the probe is retracted within the distal introducer portion and housing 322, the linear actuator 326 interacts (either directly or indirectly) with the wheel member 320 such that the posts 328A, 328B are pulled proximally away from the connector member 330, thus enabling the proximal clip portions 331A, 331B of the connector member 330 to be pinched so as to allow for release the distal clip portions 332A, 332B from engagement with the needleless access connector 334 to allow removal of the fluid transfer device 320 from engagement with the needleless access connector 334. On the other hand, when the wheel member 324 is rotated toward a second position such that the flexible probe is advanced through the distal introducer portion, the linear actuator 326 interacts (either directly or indirectly) with the wheel member 320 to move the posts 328A, 328B distally toward the connector member 330, thereby blocking the proximal clip portions 331A, 331B from being pinched inwardly and preventing the removal of the fluid transfer device 320 from the needleless access connector 334 when the probe is in an "advanced" position.

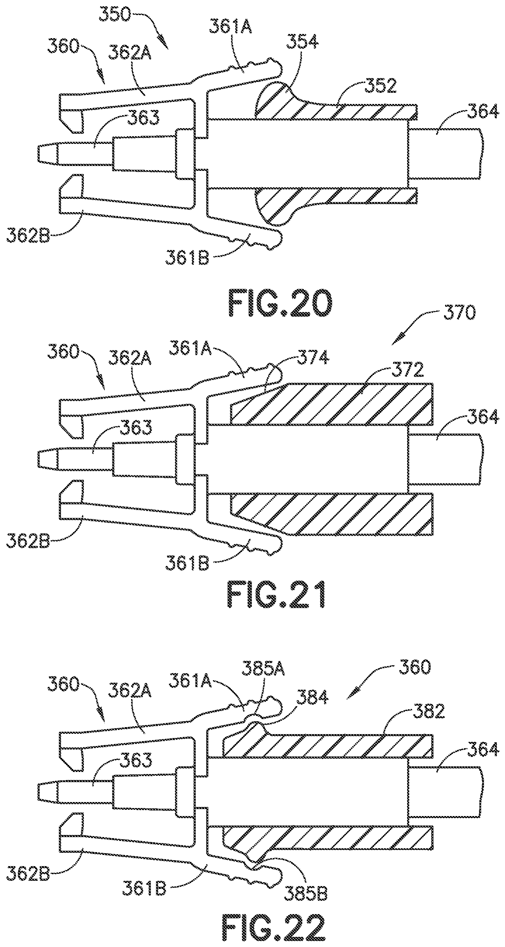

Referring to FIGS. 20-26, various interfaces between the connector member and the "blocking" feature configured to restrict use of the connector member are illustrated in accordance with aspects of the present disclosure. First, FIG. 20 shows a portion of a fluid transfer device 350 having a housing 364, a distal introducer portion 363, and a connector member 360. Connector member 360 includes opposing distal clip portions 362A, 362B allowing for securement of the fluid transfer device 350 to a surface of the needleless access connector, and a pair of proximal clip portions 361A, 361B being sized and configured to be pinched by a user to release the connector member 360. A locking member 352 is provided with fluid transfer device, with the locking member 352 having projecting portions 354 capable of blocking (or substantially blocking) the proximal clip portions 361A, 361B from being pinched in certain configurations. In some embodiments, the locking member 352 may be configured as a ring, and may be symmetrical. In other embodiments, the locking member may be asymmetrical, and/or may only block one of the two proximal clip portions.

FIG. 21 illustrates a fluid transfer device 370 in accordance with another embodiment, wherein the device includes a locking member having a linear portion 372 and one or more angled faces 374, wherein the angled face(s) 374 are configured to mimic the shape of a corresponding surface of the proximal clip portions 361A, 361B.

Next, referring to FIG. 22, a fluid transfer device 380 in accordance with another embodiment is illustrated. Fluid transfer device 380 includes a locking member having a linear portion 382 and one or more projections 384, with the projection(s) 384 being positioned, sized, and configured to mate a corresponding indent 385A, 385B of the respective proximal clip portions 361A, 361B.

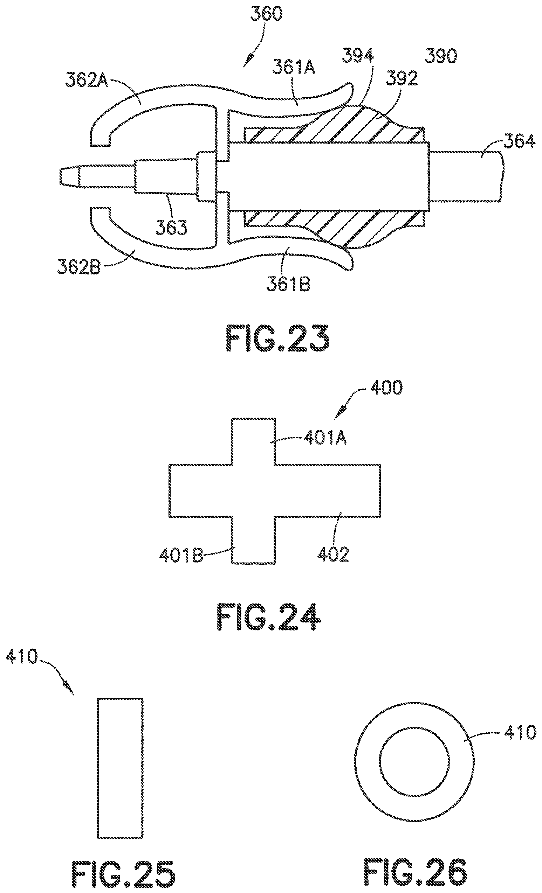

FIG. 23 illustrates a fluid transfer device 390 in accordance with another embodiment, wherein the device includes a locking member having a linear portion 392 and one or more curved faces 394, wherein the curved face(s) 394 are configured to mimic the shape of a corresponding curved inner surface of proximal clip portions 361A, 361B.

Referring to FIG. 24, a locking member 400 in accordance with another aspect of the present disclosure is illustrated. Locking member 400 is not ring-shaped, but instead includes a pair of opposing posts 401A, 401B extending from a linear portion 402 in order to selectively block movement of the proximal clip portions (not shown).

Finally, FIGS. 25 and 26 illustrate a locking member 410 that is configured as a ring-shaped member. It is to be understood that locking member 410 may be configured to extend around a distal end portion of the housing 364 so as to selectively block actuation of the connector member 360.

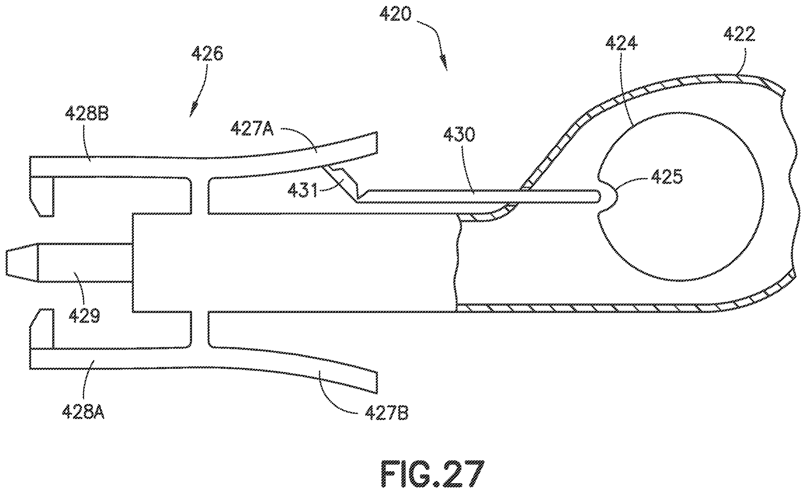

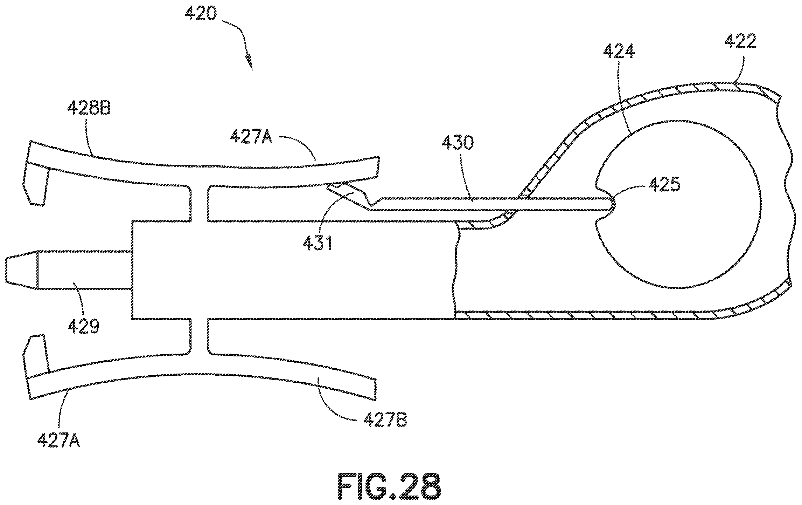

Next, referring to FIGS. 27 and 28, a fluid transfer device 420 in accordance with another aspect of the present disclosure is illustrated. The fluid transfer device 420 includes a housing 422 and a wheel member 424. Wheel member 424 is configured to interact with an elongated, flexible probe (not shown) to advance and retract the probe through a distal introducer portion 429 located on a distal end portion of the housing 422. A connector member 426 is also provided, with connector member 426 being configured for selectively coupling the fluid transfer device 420 to, e.g., a needleless access connector of an adaptor coupled to the vascular access device (not shown). Connector member 426 is configured as an alligator clip-type connector, with opposing distal clip portions 428A, 428B allowing for securement of the fluid transfer device 420 to a surface of the needleless access connector, and a pair of proximal clip portions 427A, 427B being sized and configured to be pinched or otherwise manipulated by a user to release the connector member 420 from engagement with the needleless access connector.

The fluid transfer device 420 further includes a locking mechanism comprising at least one living hinge member 431 coupled at a distal end thereof to at least one of proximal clip portions 427A, 427B. The living hinge member(s) 431 include a proximal end portion 430, with both the living hinge member 431 and proximal end portion 430 being movable upon inward movement of the proximal clip portions 427A, 427B of the connector member 426.

Furthermore, the wheel member 424 includes a slot 425 provided on a radial surface thereof. The slot 425 is sized and configured to accommodate at least a portion of the proximal end portion 430 of the locking mechanism when wheel member 424 is in certain orientations. For example, as shown in FIGS. 27 and 28, when the wheel member 424 is rotated to a first position such that the probe is retracted within the distal introducer portion 429 and housing 422, the proximal end portion 430 of the locking mechanism is substantially aligned with the slot 425 formed in the wheel member 424. Accordingly, when the proximal clip portions 427A, 427B of the connector member 426 are pinched, the living hinge member(s) 431 pivot to direct the proximal end portion 430 into the slot 425, thereby allowing the proximal clip portions 427A, 427B to be fully pinched so as to allow for release the distal clip portions 428A, 428B from engagement with the needleless access connector and, thus, enable removal of the fluid transfer device 420 from engagement with the needleless access connector. Additionally, the spring-like action of the connector member 426 also enables the proximal end portion 430 to retract away from the slot 425 when the proximal clip portions 427A, 427B are released by the user, as the living hinge member(s) 431 is pulled distally when the proximal clip portions 427A, 427B are released.

Conversely, while not shown, it is to be understood that when the wheel member 424 is rotated toward a second position such that the flexible probe is advanced through the distal introducer portion 429, the proximal end portion 430 of the locking mechanism is out of alignment with the slot 425 formed in the wheel member 424. In this position, when the proximal clip portions 427A, 427B of the connector member 426 are pinched, the living hinge member(s) 431 pivot to direct the proximal end portion 430 toward the wheel member 424, but the proximal end portion 430 does not enter the slot 425 and is instead blocked by the radial surface of the wheel member 424, which prevents the proximal clip portions 427A, 427B from being pinched so as to allow for release the distal clip portions 428A, 428B. Accordingly, in this "advanced" position of the wheel member 424, the locking mechanism comprising the living hinge member(s) 431 and proximal end portion 430 interact to substantially prevent inward motion (or pinching) of the proximal clip portions 427A, 427B, thus preventing removal of the fluid transfer device 420 from the needleless access connector when the probe is in an "advanced" position.

While the locking mechanism described above and shown in FIGS. 27 and 28 comprises a living hinge member 431 and proximal end portion 430, it is to be understood that the locking mechanism is not limited as such, and may include, e.g., multi-part hinges to replace the living hinge(s), etc.

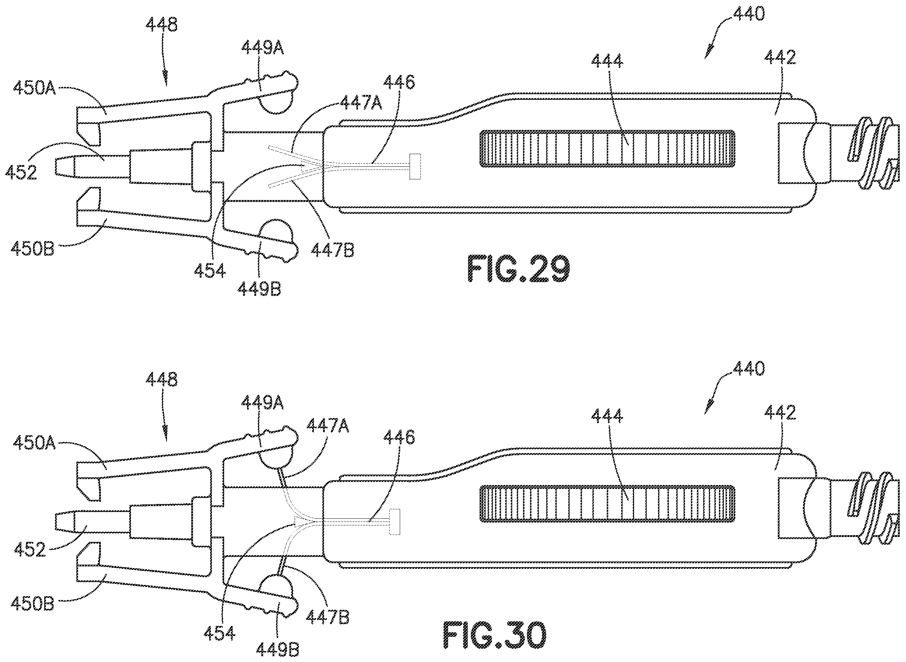

Referring now to FIGS. 29 and 30, a fluid transfer device 440 in accordance with another aspect of the present disclosure is illustrated. The fluid transfer device 440 includes a housing 442 and a wheel member 444. Wheel member 444 is configured to interact with an elongated, flexible probe (not shown) to advance and retract the probe through a distal introducer portion 452 located on a distal end portion of the housing 442. A connector member 448 is also provided, with connector member 448 being configured for selectively coupling the fluid transfer device 440 to, e.g., a needleless access connector of an adaptor coupled to the vascular access device (not shown). Connector member 448 is configured as an alligator clip-type connector, with opposing distal clip portions 450A, 450B allowing for securement of the fluid transfer device 440 to a surface of the needleless access connector, and a pair of proximal clip portions 449A, 449B being sized and configured to be pinched or otherwise manipulated by a user to release the connector member 440 from engagement with the needleless access connector.

The fluid transfer device 420 further includes a locking mechanism comprising a proximal arm member 446 and a pair of distal arm members 447A, 447B pivotally coupled to the proximal arm member 446. The proximal arm member 446 is coupled to the wheel member 444 by any appropriate mechanism (e.g., a rack-and-pinion mechanism, a worm gear mechanism, etc.) such that rotation of the wheel member 444 in either direction results in longitudinal displacement of proximal arm member 446. Additionally, the distal arm members 447A, 447B are configured to pivot relative to the proximal arm member 446 dependent upon the longitudinal position of the proximal arm member 446. That is, when the proximal arm member 446 is displaced proximally, the distal arm members 447A, 447B are configured to pivot inward (i.e., closer to one another), while when the proximal arm member 446 is displaced distally, the distal arm members 447A, 447B are configured to pivot outward.

Referring to FIG. 29, when the wheel member 444 is rotated to a first position such that the probe is retracted within the distal introducer portion 452 and housing 442, the proximal arm member 446 is pulled proximally away from the connector member 448, thereby pivoting the distal arm members 447A, 447B inward relative to one another. Accordingly, when the proximal clip portions 449A, 449B of the connector member 448 are pinched, the distal arm members 449A, 449B do not act as a block, thereby allowing the proximal clip portions 449A, 449B to be fully actuated so as to allow for release the distal clip portions 450A, 450B from engagement with the needleless access connector and, thus, enable removal of the fluid transfer device 440 from engagement with the needleless access connector.

On the other hand, referring to FIG. 30, when the wheel member 444 is rotated toward a second position such that the flexible probe is advanced through the distal introducer portion 452, the proximal arm member 446 is pushed distally toward the connector member 448, thereby pivoting the distal arm members 447A, 447B outward relative to one another. In some embodiments, a wedge or ramp 454 is provided, with the distal arm members 447A, 447B being forced outward when contacting ramp 454 as the proximal arm member 446 is pushed distally. Alternatively, in other embodiments, one or more springs may be coupled to the respective distal arm members 447A, 447B so as to force the arm members 447A, 447B outward when the proximal arm member 446 is pushed distally. In this position, when the proximal clip portions 449A, 449B of the connector member 448 are pinched, the distal arm members 447A, 447B act as a block to prevent the proximal clip portions 449A, 449B from being pinched so as to allow for release the distal clip portions 450A, 450B. Accordingly, in this "advanced" position of the wheel member 444, the locking mechanism comprising the proximal arm member 446 and pair of distal arm members 447A, 447B interact to substantially prevent inward motion (or pinching) of the proximal clip portions 449A, 449B, thus preventing removal of the fluid transfer device 440 from the needleless access connector when the probe is in an "advanced" position.

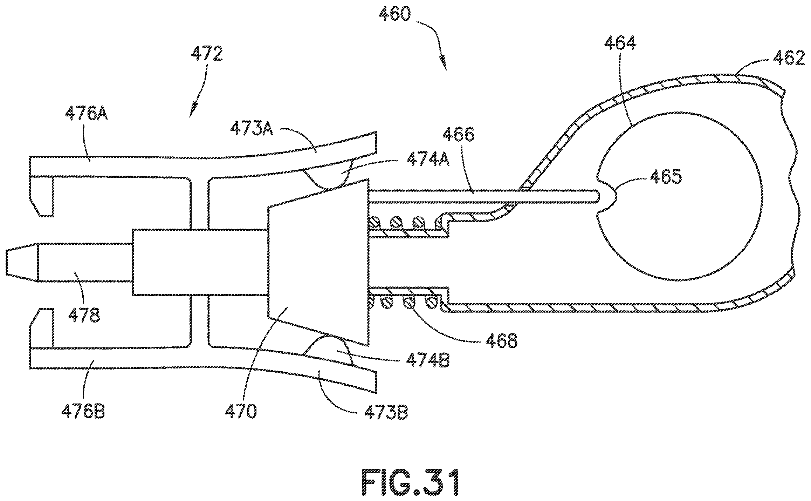

Referring now to FIG. 31, a fluid transfer device 460 in accordance with another aspect of the present disclosure is illustrated. The fluid transfer device 460 includes a housing 462 and a wheel member 464. Wheel member 464 is configured to interact with an elongated, flexible probe (not shown) to advance and retract the probe through a distal introducer portion 478 located on a distal end portion of the housing 462.

A connector member 472 is also provided, with connector member 472 being configured for selectively coupling the fluid transfer device 460 to, e.g., a needleless access connector of an adaptor coupled to the vascular access device (not shown). Connector member 472 is configured as an alligator clip-type connector, with opposing distal clip portions 476A, 476B allowing for securement of the fluid transfer device 460 to a surface of the needleless access connector, and a pair of proximal clip portions 473A, 473B being sized and configured to be pinched or otherwise manipulated by a user to release the connector member 460 from engagement with the needleless access connector. In some embodiments, the proximal clip portions 473A, 473B may include respective inner projections 474A, 474B.

The fluid transfer device 460 further includes a locking mechanism comprising a ramped distal member 470 and an arm member 466, with the ramped distal member 470 biased distally via a biasing member 468 (e.g., a spring). Ramped faces of the ramped distal member 470 are configured to interact with at least the inner projections 474A, 474B of the proximal clip portions 473A, 473B so as to displace the ramped distal member 470 and arm member 466 proximally upon inward movement of the proximal clip portions 473A, 473B of the connector member 472 in some configurations, as will be discussed further below.

The wheel member 464 includes a slot 465 provided on a radial surface thereof. The slot 465 is sized and configured to accommodate at least a portion of the arm member 466 of the locking mechanism when wheel member 464 is in certain orientations. For example, when the wheel member 464 is rotated to a first position such that the probe is retracted within the distal introducer portion 478 and housing 462, at least a proximal portion of the arm member 466 of the locking mechanism is substantially aligned with the slot 465 formed in the wheel member 464. Accordingly, when the proximal clip portions 473A, 473B of the connector member 472 are pinched, the ramped distal member 470 is displaced proximally in resistance to the biasing force of the biasing member 468, with the arm member 466 also being displaced proximally such that at least a portion of the arm member 466 enters the slot 465 of wheel member 464. Such a configuration allows the proximal clip portions 473A, 473B to be fully pinched so as to allow for release the distal clip portions 476A, 476B from engagement with the needleless access connector and, thus, enable removal of the fluid transfer device 460 from engagement with the needleless access connector.

Conversely, while not shown, it is to be understood that when the wheel member 464 is rotated toward a second position such that the flexible probe is advanced through the distal introducer portion 478, the arm member 466 of the locking mechanism is out of alignment with the slot 465 formed in the wheel member 464. In this position, when the proximal clip portions 473A, 473B of the connector member 472 are pinched, the ramped distal member 470 is unable to be displaced proximally in resistance to the biasing force of the biasing member 468, as the arm member 466 of the locking mechanism does not enter the slot 465, as it is instead blocked by the radial surface of the wheel member 464. This blocking of the ramped distal member 470 from proximal displacement prevents the proximal clip portions 473A, 473B from being fully pinched so as to allow for release the distal clip portions 476A, 476B. Accordingly, in this "advanced" position of the wheel member 464, the locking mechanism comprising the ramped distal member 470 and the arm member 466 interact to substantially prevent inward motion (or pinching) of the proximal clip portions 473A, 473B, thus preventing removal of the fluid transfer device 460 from the needleless access connector when the probe is in an "advanced" position.

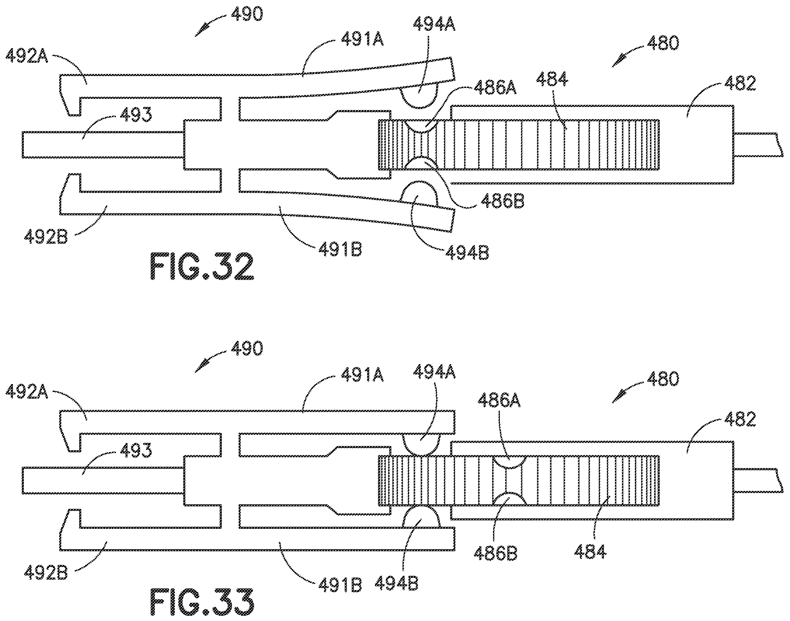

Next, referring to FIGS. 32 and 33, a fluid transfer device 480 in accordance with another aspect of the present disclosure is illustrated. The fluid transfer device 480 includes a housing 482 and a wheel member 484. Wheel member 484 is configured to interact with an elongated, flexible probe (not shown) to advance and retract the probe through a distal introducer portion 493 located on a distal end portion of the housing 482. A connector member 490 is also provided, with connector member 490 being configured for selectively coupling the fluid transfer device 480 to, e.g., a needleless access connector of an adaptor coupled to the vascular access device (not shown). Connector member 490 is configured as an alligator clip-type connector, with opposing distal clip portions 492A, 492B allowing for securement of the fluid transfer device 480 to a surface of the needleless access connector, and a pair of proximal clip portions 491A, 491B being sized and configured to be pinched or otherwise manipulated by a user to release the connector member 490 from engagement with the needleless access connector. The proximal clip portions 491A, 491B include respective inner projections 494A, 494B.

Unlike the fluid transfer devices described above with respect to FIGS. 2-31, which included distinct locking mechanisms to selectively prevent actuation of the connector member, fluid transfer device 480 relies upon direct interaction between the proximal clip portions 491A, 491B and the wheel member 484 in order to selectively prevent (or allow) for actuation of the connector member. Specifically, as shown in FIGS. 32 and 33, the wheel member 484 includes a pair of slots 486A, 486B provided on opposing side surfaces thereof. In other embodiments, it is to be understood that the slots 486A, 486B may instead be configured as indentations or a single through-hole extending through the wheel member 484.

Each slot 486A, 486B is sized and configured to accommodate at least a portion of the inner projections 494A, 494B of the wheel member 484 when the wheel member 484 is in a particular orientation. For example, referring to FIG. 32, when the wheel member 484 is rotated to a first position such that the probe is retracted within the distal introducer portion 493 and housing 482, the inner projections 494A, 494B are substantially aligned with the slots 486A, 486B formed in the wheel member 484. Accordingly, when the proximal clip portions 491A, 491B of the connector member 190 are pinched, the inner projections 494A, 494B are able to at least partially enter the slots 486A, 486B. Such a configuration allows the proximal clip portions 491A, 491B to be fully pinched so as to allow for release the distal clip portions 492A, 492B from engagement with the needleless access connector and, thus, enable removal of the fluid transfer device 180 from engagement with the needleless access connector.

However, referring to FIG. 33, when the wheel member 484 is rotated toward a second position such that the flexible probe is advanced through the distal introducer portion 493, the inner projections 494A, 494B become out of alignment with the slots 486A, 486B formed in the wheel member 484. In this position, when the proximal clip portions 491A, 491B of the connector member 490 are pinched, the inner projections 494A, 494B are blocked by the opposing side surfaces of the wheel member 484, thereby preventing the proximal clip portions 491A, 491B from being fully pinched so as to allow for release the distal clip portions 492A, 492B. Accordingly, in this "advanced" position of the wheel member 484, the connector 490 and the wheel member 484 interact to substantially prevent inward motion (or pinching) of the proximal clip portions 491A, 491B, thus preventing removal of the fluid transfer device 480 from the needleless access connector when the probe is in an "advanced" position.

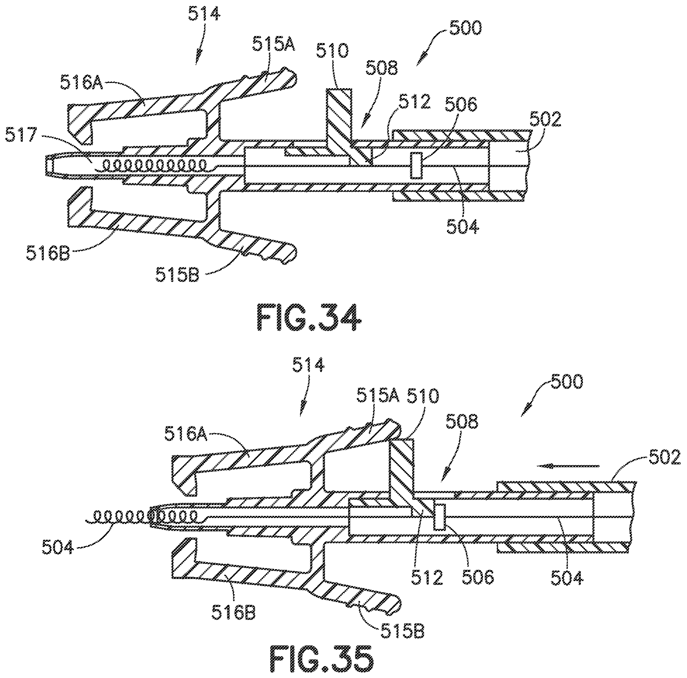

Referring now to FIGS. 34 and 35, a fluid transfer device 500 in accordance with another aspect of the present disclosure is illustrated. The fluid transfer device 500 includes a housing 502 and a probe advancement mechanism (not shown) comprising, e.g., a wheel member, a linear advancing member, etc. The probe advancement mechanism is configured to operatively advance and/or retract a flexible probe 504 to and from a distal introducer portion 517 located on a distal end portion of the housing 500.

A connector member 514 is also provided, with connector member 514 being configured for selectively coupling the fluid transfer device 500 to, e.g., a needleless access connector of an adaptor coupled to the vascular access device (not shown). Connector member 514 is configured as an alligator clip-type connector, with opposing distal clip portions 516A, 516B allowing for securement of the fluid transfer device 500 to a surface of the needleless access connector, and a pair of proximal clip portions 515A, 515B being sized and configured to be pinched or otherwise manipulated by a user to release the connector member 514 from engagement with the needleless access connector.

Unlike the fluid transfer devices described above with respect to FIGS. 2-33, which utilized interaction with the probe advancement mechanism (e.g., a wheel member) in order to selectively prevent disengagement of the connector member, fluid transfer device 500 utilizes the probe 504 itself to control prevention of disengagement of the connector member. In particular, probe 504 includes a projecting member 506 extending from at least one side of the probe 504 at a location proximal to the connector member 514. The projecting member 506 may be integrally formed as part of the probe 504, or may be a separate component coupled to the probe 504 via any appropriate connection method.

The fluid transfer device 500 further includes a locking member 508, wherein locking member 508 is configured to move longitudinally along the housing 502, with the longitudinal movement being based upon the position of the probe 504. Locking member 508 includes a proximal end portion 512 and a projecting portion 510. The proximal end portion 512 is configured to provide a contact surface between the locking member and the projection member 506 as the probe 504 is advanced from the distal introducer portion 517. While not shown, it is to be understood that the locking member 508 may be biased in the proximal direction (i.e., away from the connector member 514) via, e.g., a spring.

As shown in FIG. 34, when the probe 504 is in a first position such that the probe 504 is retracted within the distal introducer portion 517 and housing 502, the projecting member 506 does not contact the proximal end portion 512 of the locking member 508, thereby allowing the locking member 508 to be biased toward its proximal-most position. Accordingly, when the proximal clip portions 515A, 515B of the connector member 514 are pinched, one or more of the proximal clip portions 515A, 515B do not come into contact with the projecting portion 510 of the locking member 508, allowing the proximal clip portions 515A, 515B to be fully pinched so as to allow for release the distal clip portions 516A, 516B from engagement with the needleless access connector and, thus, enable removal of the fluid transfer device 500 from engagement with the needleless access connector.

On the other hand, referring to FIG. 35, when the probe 504 is advanced toward a second position so as to be advanced through the distal introducer portion 517, the projecting member 506 is configured to push the locking member 508 distally, thereby aligning the projecting portion 510 of the locking member 508 substantially below one or more of the proximal clip portions 515A, 515B. In this position, when the proximal clip portions 515A, 515B of the connector member 514 are pinched, the projecting portion 510 prevents the proximal clip portions 515A, 515B from being fully pinched. Accordingly, in this "advanced" position of the probe 504, the locking mechanism 508 acts to substantially prevent inward motion (or pinching) of the proximal clip portions 515A, 515B, thus preventing removal of the fluid transfer device 500 from the needleless access connector.

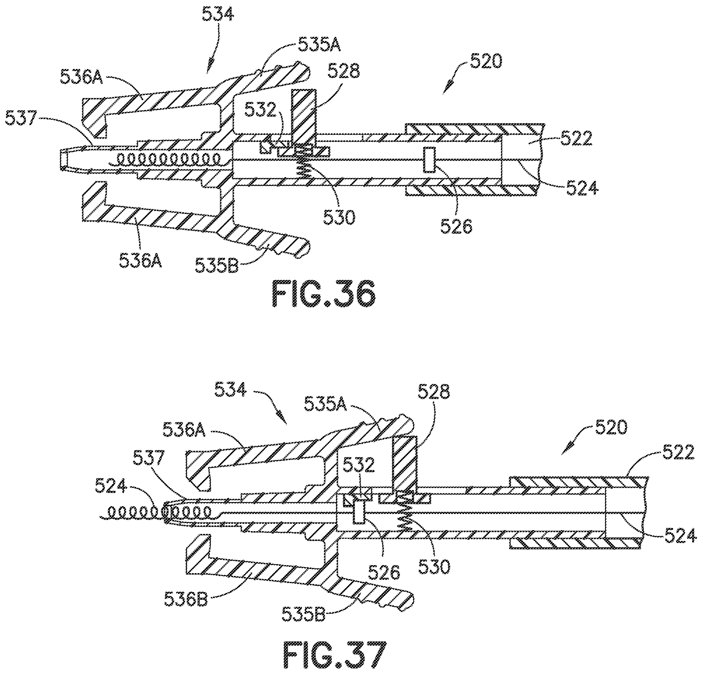

Next, referring to FIGS. 36 and 37, a fluid transfer device 520 in accordance with another aspect of the present disclosure is illustrated. The fluid transfer device 520 includes a housing 522 and a probe advancement mechanism (not shown) comprising, e.g., a wheel member, a linear advancing member, etc. The probe advancement mechanism is configured to operatively advance and/or retract a flexible probe 524 to and from a distal introducer portion 537 located on a distal end portion of the housing 520.

A connector member 534 is also provided, with connector member 534 being configured for selectively coupling the fluid transfer device 520 to, e.g., a needleless access connector of an adaptor coupled to the vascular access device (not shown). Connector member 534 is configured as an alligator clip-type connector, with opposing distal clip portions 536A, 536B allowing for securement of the fluid transfer device 520 to a surface of the needleless access connector, and a pair of proximal clip portions 535A, 535B being sized and configured to be pinched or otherwise manipulated by a user to release the connector member 534 from engagement with the needleless access connector.

Similar to fluid transfer device 500 described above with respect to FIGS. 34 and 35, fluid transfer device 520 utilizes the probe 524 itself to control prevention of disengagement of the connector member 534. In particular, probe 524 includes a projecting member 526 extending from at least one side of the probe 524 at a location proximal to the connector member 534. The projecting member 526 may be integrally formed as part of the probe 524, or may be a separate component coupled to the probe 524 via any appropriate connection method.

The fluid transfer device 520 further includes a locking member 528, wherein locking member 528 is configured to move laterally with respect to the housing 522 in a direction substantially transverse to the direction of the probe 524. Lateral movement of the locking member 528 is based upon the position of the probe 524, with the locking member 528 capable of being biased radially outwardly by a biasing member 530 (e.g., a spring). The biasing member 530 may be offset so as to avoid interference with the probe 524 within the housing 522.

As shown in FIG. 36, when the probe 524 is in a first position such that the probe 524 is retracted within the distal introducer portion 537 and housing 522, the projecting member 526 does not contact any portion of the locking member 528, nor does the projecting member 526 contact a holding member 532 associated with the locking member 528. Accordingly, when the proximal clip portions 535A, 535B of the connector member 534 are pinched, one or more of the proximal clip portions 535A, 535B do not come into contact with the locking member 528, as the holding member 532 acts to hold the locking member 528 and biasing member 530 in a compressed position within the housing 522. This position of the locking member 528 allows the proximal clip portions 535A, 535B to be fully pinched so as to allow for release the distal clip portions 536A, 536B from engagement with the needleless access connector and, thus, enable removal of the fluid transfer device 520 from engagement with the needleless access connector.

Conversely, referring to FIG. 37, when the probe 524 is advanced toward a second position so as to be advanced through the distal introducer portion 537, the projecting member 526 is configured to push the holding member 532 distally, thereby releasing the locking member 528 from engagement with the holding member 532 such that the biasing member 530 biases the locking member 528 radially outward to align at least a portion of the locking member 528 substantially below one or more of the proximal clip portions 535A, 535B. In this position, when the proximal clip portions 535A, 535B of the connector member 534 are pinched, the locking member 528 prevents one or both of the proximal clip portions 535A, 535B from being fully pinched, thereby preventing removal of the fluid transfer device 520 from the needleless access connector.

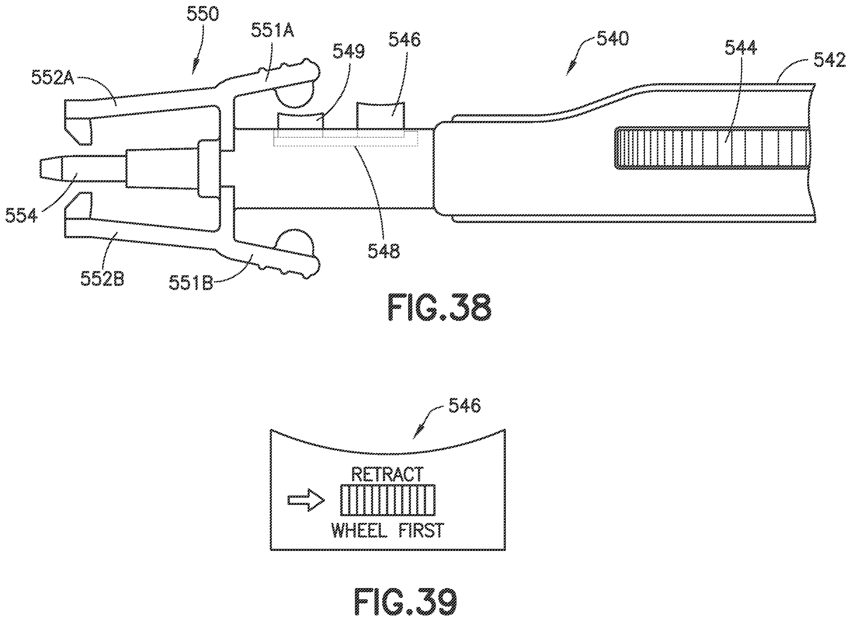

Referring now to FIGS. 38 and 39, a fluid transfer device 540 in accordance with another aspect of the present disclosure is illustrated. The fluid transfer device 540 includes a housing 542 and a probe advancement mechanism 544 comprising, e.g., a wheel member. The probe advancement mechanism 544 is configured to operatively advance and/or retract a flexible probe (not shown) to and from a distal introducer portion 554 located on a distal end portion of the housing 542. A connector member 550 is also provided, with connector member 550 being configured for selectively coupling the fluid transfer device 540 to, e.g., a needleless access connector of an adaptor coupled to the vascular access device (not shown). Connector member 550 is configured as an alligator clip-type connector, with opposing distal clip portions 552A, 552B allowing for securement of the fluid transfer device 540 to a surface of the needleless access connector, and a pair of proximal clip portions 551A, 551B being sized and configured to be pinched or otherwise manipulated by a user to release the connector member 550 from engagement with the needleless access connector.

The fluid transfer device further comprises a locking member 548 positioned on the housing 542 near the connector member 550 so as to be longitudinally slidable along the housing 542. The locking member 548 may include a proximal button portion 546 configured as an interface for, e.g., a user's thumb or finger. The locking member 548 may also include a distal blocking portion 549 extending therefrom.

The locking member 548 may be biased to a default position such that the distal blocking portion 549 is positioned below one or both of the proximal clip portions 551A, 551B, thereby preventing actuation (i.e., pinching) of the proximal clip portions 551A, 551B upon insertion of the fluid transfer device 540. Only after the user manually retracts the locking member 548 by pulling the proximal button portion 546 in a proximal direction does the distal blocking portion 549 move away from the connector member 550, thereby allowing the proximal clip portions 551A, 551B to be pinched or otherwise manipulated by a user to release the connector member 550 from engagement with the needleless access connector.

As shown in FIG. 39, in some embodiments, the proximal button portion 546 (and/or other portions of the locking member 548) may include written instructions(s) or other visual indicators so as to remind the user that the probe must be retracted (via rotation of, e.g., the probe advancement mechanism 544) prior to disengagement of the locking member 548 and retraction of the fluid transfer device 540 from the needleless access connector.

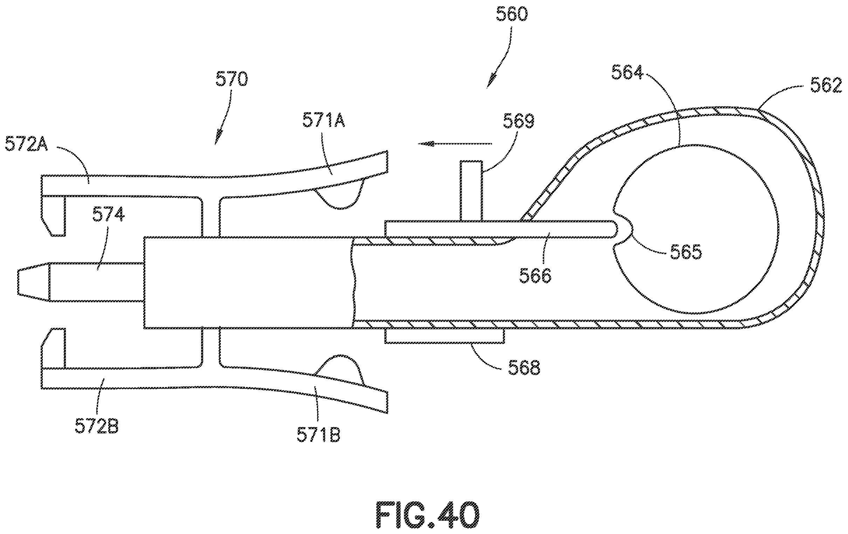

Next, referring to FIG. 40, a fluid transfer device 560 in accordance with another aspect of the present disclosure is shown. The fluid transfer device 560 includes a housing 562 and a wheel member 564. The wheel member 564 is configured to operatively advance and/or retract a flexible probe (not shown) to and from a distal introducer portion 574 located on a distal end portion of the housing 562.

A connector member 570 is also provided, with connector member 570 being configured for selectively coupling the fluid transfer device 560 to, e.g., a needleless access connector of an adaptor coupled to the vascular access device (not shown). Connector member 570 is configured as an alligator clip-type connector, with opposing distal clip portions 572A, 572B allowing for securement of the fluid transfer device 560 to a surface of the needleless access connector, and a pair of proximal clip portions 571A, 571B being sized and configured to be pinched or otherwise manipulated by a user to release the connector member 570 from engagement with the needleless access connector.

The fluid transfer device further comprises a sliding lock member 568 positioned on or within the housing 562 near the connector member 570 so as to be longitudinally slidable along a portion of the housing 562. The sliding locking member 568 may include at least one projecting portion 569 configured as an interface for, e.g., a user's thumb or finger. The sliding locking member 568 may also include an arm member 566 extending proximally therefrom.

The wheel member 564 includes a slot 565, with slot 565 sized and configured to selectively receive at least a portion of the arm member 566 therein. The portion of the arm member 566 is aligned and receivable within the slot 565 only when the wheel member 564 is rotated such that the probe is retracted within the distal introducer portion 574 and the housing 562. As such, when the arm member 566 and the slot 565 are aligned, the user may manually retract the sliding locking member 568 in a proximal direction, which enables the pair of proximal clip portions 571A, 571B to be pinched in order to release the connector member 570 from engagement with the needleless access connector. With the arm member 566 at least partially within the slot 565 in this position, the wheel member 564 is prevented from rotating, thereby preventing advancement or retraction of the probe when the connector member 570 is capable of disconnection.

However, when the user wishes to advance or retract the probe via rotation of the wheel member 564, the user must move the sliding locking member 568 distally such that the arm member 566 disengages with the slot 565. This distal movement of the sliding locking member 568 also a distal end "ring" portion of the sliding locking member 568 substantially below one or more of the proximal clip portions 571A, 571B, thereby preventing actuation of the connector member 570 and removal of the fluid transfer device 560 from the needleless access connector when the wheel member 564 is capable of rotation and, thus, while the probe may be in an advanced state beyond the distal introducer portion 574.

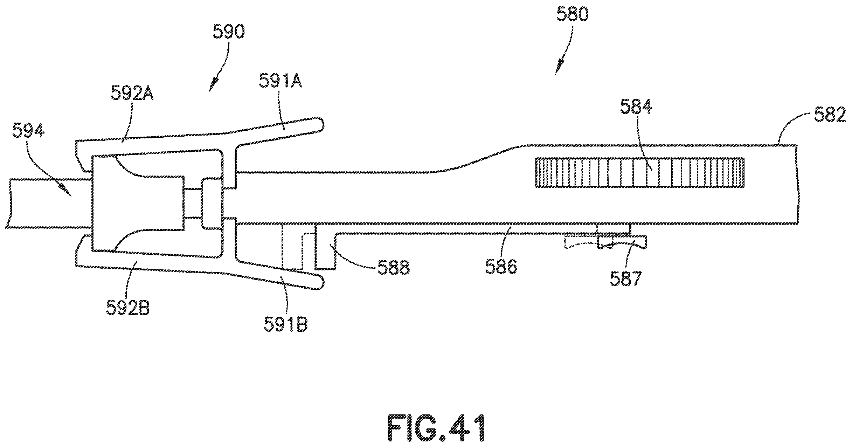

Referring now to FIG. 41, a fluid transfer device 580 in accordance with another aspect of the present disclosure is shown. The fluid transfer device 580 includes a housing 582 and a wheel member 584 configured to operatively advance and/or retract a flexible probe (not shown) to and from a distal introducer portion (not shown) located on a distal end portion of the housing 582. A connector member 590 is also provided, with connector member 590 being configured for selectively coupling the fluid transfer device 580 to, e.g., a needleless access connector 594 of an adaptor coupled to the vascular access device (not shown). Connector member 590 is configured as an alligator clip-type connector, with opposing distal clip portions 592A, 592B allowing for securement of the fluid transfer device 580 to a surface of the needleless access connector 594, and a pair of proximal clip portions 591A, 591B being sized and configured to be pinched or otherwise manipulated by a user to release the connector member 580 from engagement with the needleless access connector 594.

The fluid transfer device 580 further includes a locking member 586 positioned on or partially within the housing 582 between the connector member 590 and the wheel member 584, wherein the locking member 586 is configured to be longitudinally slidable along the housing 582. The locking member 586 may include a proximal button portion 587 configured as an interface for, e.g., a user's thumb or finger, as well as a distal blocking portion 588 extending therefrom.

While not shown in FIG. 41, the wheel member 584 may include a slot or other interface capable of interlocking with a portion of the locking member 586. That is, a portion of the locking member 586 may be aligned and receivable within the interlocking interface with the wheel member 584 only when the wheel member 584 is rotated such that the probe is retracted within the distal introducer portion and the housing 582. Accordingly, when the locking member 586 is proximally retracted such that there is an interlock between the locking member 586 and the wheel member 584, the distal blocking portion 588 of the locking member 586 is also moved proximally away from the connector member 590, which allows the pair of proximal clip portions 591A, 591B to be pinched in order to release the connector member 590 from engagement with the needleless access connector 594. Furthermore, the interlock between the locking member 586 and the wheel member 584 prevents the wheel member 584 from rotating, thereby preventing advancement or retraction of the probe when the connector member 590 is capable of disconnection from the needleless access connector 594.

Conversely, when the user wishes to advance or retract the probe via rotation of the wheel member 584, the user must move the locking member 586 distally such that the interlock between the locking member 586 and the wheel member 584 is released. This distal movement of the locking member 586 also moves the at least one projecting portion 588 substantially below one or more of the proximal clip portions 591A, 591B, thereby preventing actuation of the connector member 590 and removal of the fluid transfer device 580 from the needleless access connector 594 when the wheel member 584 is capable of rotation and, thus, while the probe may be in an advanced state beyond the distal introducer portion.

While not shown in FIG. 41, in some embodiments, it is to be understood that the proximal button portion 587 (and/or other portions of the locking member 586) may include written instructions(s) or other visual indicators so as to remind the user that the probe must be retracted (via rotation of, e.g., the probe advancement mechanism 584) prior to disengagement of the locking member 586 and removal of the fluid transfer device 580 from engagement with the needleless access connector 594.

All examples and conditional language recited herein are intended for pedagogical objects to aid the reader in understanding the invention and the concepts contributed by the inventor to furthering the art, and are to be construed as being without limitation to such specifically recited examples and conditions. Although embodiments of the present inventions have been described in detail, it should be understood that the various changes, substitutions, and alterations could be made hereto without departing from the spirit and scope of the invention.

### FURTHER ASPECTS

Aspect 1. A fluid transfer device for delivery of a probe to a patient's vascular system, the fluid transfer device comprising:
a housing;
a distal introducer portion positioned at a distal end portion of the housing and configured to penetrate a needleless access connector of a vascular access device;
a wheel member rotatable with respect to the housing, wherein the wheel member is operably coupled to the probe to advance and retract the probe through the distal introducer portion based on direction of rotation of the wheel member;
a connector member positioned proximate the distal introducer portion and configured for releasable connection to at least one surface of the needleless access connector, wherein the connector member comprises a pair of opposing distal clip portions and a pair of opposing proximal clip portions; and
a locking member, wherein the locking member is operably coupled to the wheel member and is configured to prevent actuation of at least one of the pair of opposing proximal clip portions when the wheel member is rotated to a first position and to allow actuation of at least one of the pair of opposing proximal clip portions when the wheel member is rotated to a second position.

Aspect 2. The fluid transfer device of aspect 1, wherein the wheel member comprises a clocking member, and wherein the locking member is operably coupled to the clocking member.

Aspect 3. The fluid transfer device of aspect 2, wherein the locking member comprises an arm coupled to the clocking member.

Aspect 4. The fluid transfer device of aspect 3, wherein one end of the arm comprises a post configured to ride within a cam groove formed in the clocking member.

Aspect 5. The fluid transfer device of aspect 1, wherein the locking member comprises a ring member longitudinally slidable over at least a portion of the housing.

Aspect 6. The fluid transfer device of aspect 1, wherein the locking member comprises a biasing member configured to bias the locking member in one direction.

Aspect 7. The fluid transfer device of aspect 1, further comprising a rack member and a pinion gear, wherein the pinion gear is operably coupled to the wheel member and the rack member is coupled to the locking member so as to longitudinally displace the locking member based upon rotational direction of the wheel member.

Aspect 8. The fluid transfer device of aspect 1, wherein the locking member comprises at least one tab, and wherein the at least one tab is displaceable by a wire operably coupled to the wheel member.

Aspect 9. The fluid transfer device of aspect 1, wherein the locking member comprises at least one proximal arm member, and wherein the wheel member comprises at least one slot provided on a radial surface of the wheel member, wherein the at least one slot is sized and configured to accommodate at least a portion of the at least one proximal arm member.

Aspect 10. The fluid transfer device of aspect 9, wherein the locking member further comprises at least one living hinge coupled to at least one of the pair of opposing proximal clip portions on a first end and to the at least one proximal arm member on a second end.

Aspect 11. The fluid transfer device of aspect 9, wherein the locking member further comprises a ramped distal member.

Aspect 12. The fluid transfer device of aspect 1, wherein the locking member is manually displaceable by a user.

Aspect 13. The fluid transfer device of aspect 12, wherein the locking member comprises a distal blocking portion and a proximal button portion, wherein the proximal button portion is configured as a user interface.

Aspect 14. A fluid transfer device for needle-free delivery of a probe to a patient's vascular system, the fluid transfer device comprising:
a housing;
a distal introducer portion positioned at a distal end portion of the housing and configured to penetrate a needleless access connector of a vascular access device;
a wheel member rotatable with respect to the housing, wherein the wheel member is operably coupled to the probe to advance and retract the probe through the distal introducer portion based on direction of rotation of the wheel member;
a connector member positioned proximate the distal introducer portion and configured for releasable connection to at least one surface of the needleless access connector; and
a locking member, wherein the locking member is configured to prevent disconnection of the connector member from the needleless access connector when the wheel member is rotated to a first position and to allow disconnection of the connector member when the wheel member is rotated to a second position.

Aspect 15. The fluid transfer device of aspect 14, wherein the locking member comprises a sleeve member.

Aspect 16. The fluid transfer device of aspect 15, wherein the sleeve member is configured to extend over at least a portion of the connector member when the wheel member is rotated to the first position.

Aspect 17. The fluid transfer device of aspect 14, wherein at least a portion of the locking member is configured to extend under at least a portion of the connector member when the wheel member is in the first position.

Aspect 18. The fluid transfer device of aspect 14, wherein the wheel member further comprises a pair of opposing slots provided on opposing sides of the wheel member, and wherein the locking member comprises a pair of opposing inner projections provided on a proximal portion of the connector member, the pair of opposing inner projections configured to engage the pair of opposing slots when the wheel member is rotated to the second position.

Aspect 19. The fluid transfer device of aspect 14, further comprising a projecting member extending from at least one side of the probe, wherein the projecting member is configured to enable movement of the locking member based upon a position of the probe within the housing.

Aspect 20. The fluid transfer device of aspect 14, wherein the locking member is manually displaceable by a user, and further wherein the locking member comprises a distal blocking portion and a proximal button portion, wherein the proximal button portion is configured as a user interface.

## Claims

1. A fluid transfer device for needle-free delivery of a probe to a patient's vascular system, the fluid transfer device comprising:
a housing;
a distal introducer portion positioned at a distal end portion of the housing and configured to penetrate a needleless access connector of a vascular access device;
a wheel member rotatable with respect to the housing, wherein the wheel member is operably coupled to the probe to advance and retract the probe through the distal introducer portion based on direction of rotation of the wheel member;
a connector member positioned proximate the distal introducer portion and configured for releasable connection to at least one surface of the needleless access connector; and
a locking member, wherein the locking member is configured to prevent disconnection of the connector member from the needleless access connector when the wheel member is rotated to a first position and to allow disconnection of the connector member when the wheel member is rotated to a second position.

2. The fluid transfer device of claim 1, wherein the locking member comprises a sleeve member.

3. The fluid transfer device of claim 2, wherein the sleeve member is configured to extend over at least a portion of the connector member when the wheel member is rotated to the first position.

4. The fluid transfer device of claim 1, wherein at least a portion of the locking member is configured to extend under at least a portion of the connector member when the wheel member is in the first position.

5. The fluid transfer device of claim 1, wherein the wheel member further comprises a pair of opposing slots provided on opposing sides of the wheel member, and wherein the locking member comprises a pair of opposing inner projections provided on a proximal portion of the connector member, the pair of opposing inner projections configured to engage the pair of opposing slots when the wheel member is rotated to the second position.

6. The fluid transfer device of claim 1, further comprising a projecting member extending from at least one side of the probe, wherein the projecting member is configured to enable movement of the locking member based upon a position of the probe within the housing.

7. The fluid transfer device of claim 1, wherein the locking member is manually displaceable by a user, and further wherein the locking member comprises a distal blocking portion and a proximal button portion, wherein the proximal button portion is configured as a user interface.
